# EUROPEAN PATENT SPECIFICATION

(11) **EP 2 894 217 B1**
(45) Date of publication and mention of the grant of the patent: **03.04.2019**
(21) Application number: 15150372.9
(22) Date of filing: 07.01.2015
(51) Int. Cl.: C12M 1/107, C12M 3/00, E02D 17/04, C12M 1/00

(54) **Plant for producing biogas and process for making the same plant**
Anlage zur Herstellung von Biogas und Verfahren zur Herstellung der Anlage
Installation pour la production de biogaz et son procédé de fabrication de plantes

(30) Priority: 08.01.2014 IT MI20140008
(43) Date of publication of application: 15.07.2015
(73) Proprietor: Tenax S.p.A., 23897 Viganò (Lecco) (IT); Erckert, Christof, 39010 Bolzano (BZ) (IT)
(72) Inventor: Erckert, Christof, 39010 Bolzano (BZ) (IT)
(74) Representative: Ponzellini, Gianmarco

(56) References cited:
- WO-A1-98/38391
- DE-U1-202007 004 986
- GB-A- 2 334 739
- KR-A- 20120 044 431

## Description

### FIELD OF THE INVENTION

The present invention refers to a tank for biogas producing tank and to a process for forming the same. The tank can be for example used in an industrial field for treating urban organic waste, manure produced by farming and/or sludge/residuals of the agro-industry.

### STATE OF THE ART

Biogas is a mixture of different types of gases (50% - 80% methane) produced by an anaerobic bacterial fermentation (without oxygen) of organic residuals, particularly from waste, decomposing plants, zootechnical sludge or purification sewage, agro-industry waste. The biogas producing process comprises a step of decomposing the organic matter by some types of bacteria for producing carbon dioxide, molecular hydrogen and methane (methanation of organic compounds).

Therefore biogas, generally obtained by organic-type waste, can be used for being combusted in heating boilers, for producing electric energy or, when suitably depurated (separated from other components such as carbon dioxide and sulphur, for example), can be used as bio-methane in the automotive fields (methane cars and vehicles) .

Generally, the biogas producing plants consist of at least one anaerobic digester substantially defining a tightly-closed and insulated reservoir suitable for receiving organic matter.

Inside the anaerobic digester, the fermentation process takes place which, due to the anaerobic microorganisms present inside the reservoir itself, enables to obtain biogas and/or (solid and liquid) bio-fertilizers.

The biogas, formed inside the digester, is withdrawn and stored in a gas holder, inside which the pressure and the biogas composition are made uniform. Particular gas supplying systems enable to constantly transfer the biogas from a gas holder to the system for the combined production of: gas, thermal-electric energy.

The currently known plants comprise further preprocessing and/or storage tanks/reservoirs of the organic matter, in which the latter is respectively prepared for the digester (the organic matter is kept at a determined temperature and is mixed in order to ensure a determined uniformity) and stored at the end of the fermentation process for ensuring a full degradation of the organic matter and also the greatest exploitation of the latter.

The plants used for producing biogas differ as a function of the electric and/or thermal power which one desires to obtain; the variables enabling to determine the "size" of the plant are, actually, the number of digestion tanks (the number of digesters) and the size of such tanks. Generally, both the digestion tanks and the storage tanks have a diameter comprised between 15 and 25 meters and a height comprised between 6 and 8 meters.

For the sake of the following discussion, it is particularly interesting the structure of tanks suitable for holding organic matter and the generated biogas (structure of the digestion and storage tanks).

To date, the containing tanks are made of reinforced concrete or steel coated by fused glass.

The reinforced concrete tanks are generally formed by a cylindrical base which can be underground or is made at least partially project from the ground. The most widely used solution is embodied by tanks whose base is substantially aligned with the ground level; the only portion located below the ground is the tank foundation.

The base is made of reinforced concrete and has a diameter generally comprised between 18 and 25 meters. A cylindrical side wall made also of reinforced concrete emerges from the base: the height of the tank, represented by the vertical extension of the side wall, does not generally exceed 6 meters.

At least one covering membrane is mechanically fixed to the upper end of the base which, in combination with the base, defines a tightly-closed reservoir. The membrane is fixed by cementing a set of threaded rods at the top of the side wall, which upwardly project with respect to the latter on all the tank perimeter. The membrane is fixed by forming cuts in the latter, suitable for receiving the threaded rods (the rods are inserted into the cuts); the whole is clamped as a package with suitable closing cups which are in turn covered by further waterproof membrane patches welded along the contour: such solution would enable to prevent leakages from the inside or a penetration from the outside.

A completely analogous structure forms the steel tanks, whose only difference with respect to the reinforced concrete tanks is represented by the material used for making the base.

Despite the fact that the above described tanks enable to hold organic matter and to produce/collect biogas, such tanks are not devoid of disadvantages.

Particularly, the presently known tanks (tanks of reinforced concrete and steel) have a low flexibility: the digester and storage reservoir made of concrete or steel are considerably stiff due to their own structure and the used material; small movements due to the ground setting could be transmitted to the whole structure which, due to its low structural flexibility, could be damaged, some areas could be cracked forming in this way cracks which could lead to infiltrations, biomass loss and/or biogas loss.

A further disadvantage is due to the overall costs of the plant regarding the high costs of the materials (it is necessary a huge amount of reinforced concrete and/or steel), installation costs due to the particular problems in building the plant, and the disposal costs (due to the large quantity of material which must be disposed of once the plant ends its useful life). Further it is not excluded the impact on the surrounding landscape of these structures made of reinforced concrete or steel.

A further limitation is represented by the covering membrane fixing mechanism implementable for the structures made of reinforced concrete and/or steel: the necessity of making cuts in the covering membrane for inserting the threaded rods forms a series of weakening points in the membrane itself which can form undesired tears/breaches with a consequent loss of biogas and/or air leakages.

As for the tank structure, the way of fixing the membrane by a threaded rod, is a particularly stiff constraining system which is not capable of adapting to possible displacements and accommodations of the membrane. Further it is not excluded that the rod is an area wherein stresses acting on the membrane are concentrated; so it is necessary to provide a high number of threaded rods located above the tank side wall, such situation increase the overall costs of the plant (an increase of the material cost, installation and disposal costs).

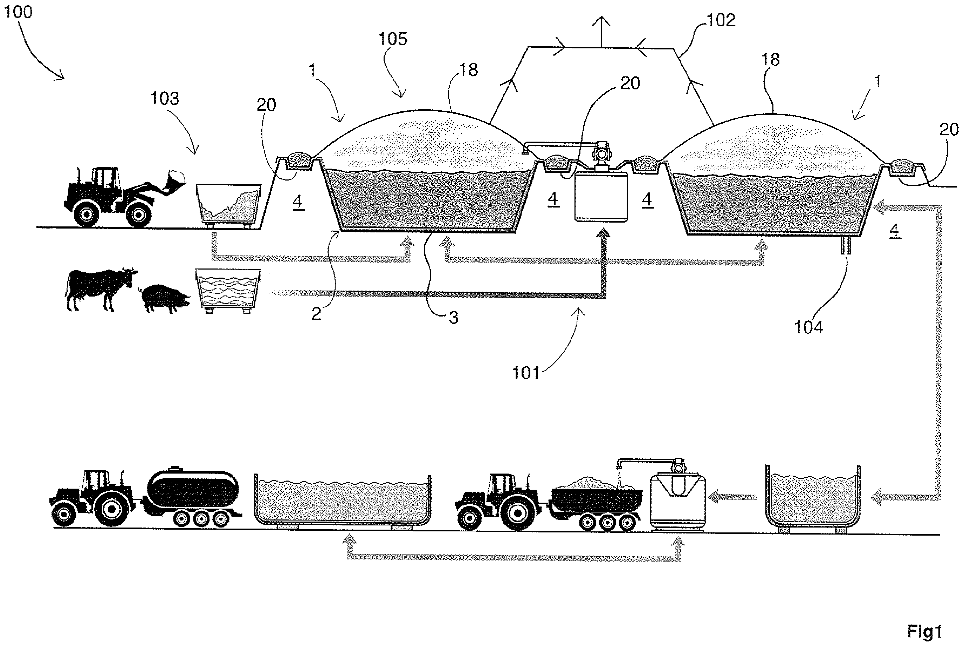

DE202007004986 discloses a biogas reactor in a sealed basin with a gas membrane and a protective membrane. A trench filled with stones keeps the membranes in place (fig. 1, [0002]).

### OBJECT OF THE INVENTION

Therefore it is an object of the present invention to substantially solve at least one of the disadvantages and/or limits of the prior solutions.

A first goal of the invention consists of providing a particularly flexible tank for biogas plants, capable of easily adapting without breaches or leakages, to possible subsidences/settings of the ground. A further main object of the invention consists of providing a tank for biogas plants having a low environmental impact on the landscape. It is a further goal of the invention to provide a tank for biogas plants which is firmly tight against leakages or infiltrations; particularly, a tank suitable for ensuring an optimal seal of a tank covering membrane without causing undesired stresses which could damage the membrane itself. It is also an object of the invention to provide a tank implementable with low cost materials in order to reduce the overall plant cost. It is also an object of the invention to provide a tank for biogas plants of simple realization which enables to reduce at a minimum the installation and disposal costs of the structure. A further object of the present invention consists of providing a process for forming a biogas plant tank which is extremely simple such to ensure a fast installation of the plant in order to reduce the cost due to the realization and disposal of the plant. One or more of the above described objects which will be better understood in the following description are substantially met by a tank for biogas producing plants and by a process for forming the same according to one or more of the attached claims.

### SUMMARY

The aspects of the invention will be described in the following.

In a 1st aspect it is provided a process for forming a tank (1) for biogas plants comprising at least the following steps:
- providing a plurality of reinforcing elements (7), each of them having a reticular structure (8), each reinforcing element (7) comprising:
   - a plurality of first elements (8a) spaced from each other and having an elongated shape according to a respective main extension direction;
   - a plurality of second elements (8b) spaced from each other, which substantially extend along a direction transversal to said first elements (8a), second elements (8b) having an elongated shape according to a respective main extension direction;
- providing a predetermined quantity of ground;
- forming a containing site (2) comprising at least one base (3) and at least one side wall (4) mainly of ground, transversally emerging from base (3), side wall (4) having an inner face (4a) which, together with the base (3), defines a containing cavity (5), having a closed outline, arranged to receive a predetermined quantity of organic matter configured for generating a biogas following an anaerobic bacterial fermentation, the containing site (2) further comprising a free edge (6) placed opposite with respect to the base (3) and delimiting an opening of the containing cavity (5);
the step of providing the side wall (4) of ground comprises at least the following sub-steps:
- forming a first ground compacted layer (25) having a rising surface (33) and an upper abutment surface (34), the upper abutment surface (34) extending around the base (3) to define a closed outline;
- positioning on the upper abutment surface (34) of the first ground layer (25), a plurality of reinforcing elements (7), said plurality of reinforcing elements (7) extending around the base (3) to define a closed outline;
- connecting to each other said reinforcing elements (7) in order to define a continuous layer of elements suitable for covering at least partially the upper abutment surface (34) of the first ground layer (25);
- positioning, after having positioned the reinforcing elements (7), a second ground layer (26) so that the reinforcing elements (7) are at least partially interposed between the first and second ground layers (25; 26), said second ground layer (26) having a respective rising surface (33) and a respective upper abutment surface (34), the upper abutment surface (34) extending around the base (3) to define a closed outline;
each ground layer and the respective reinforcing elements (7) associated to it, defining a reinforced layer (9), the process provides to repeat said sub-steps to define a plurality of reinforced layers (9) overlapping each other so that these latter define the inner surface (4a) of the side wall (4).

In a 2nd aspect according to the preceding aspect, the reticular structure (8) has a monolithic structure at least partially flexible of plastics material.

In a 3rd aspect according to anyone of the preceding aspects, the step of providing the side wall (4) provides the positioning of a plurality of reinforcing elements (7) inside the lateral wall (4) in order to define a reinforced ground wall, said reinforcing elements being located at the inner face (4a).

In a 4th aspect according to anyone of the preceding aspects, the step of positioning each reinforcing element (7) comprises at least the following steps:
- spreading on the upper abutment surface (34) a first segment (13) of the reinforcing element (7);
- raising a front portion (27) of the reinforcing element (7) to define at least a second segment (14) transversal to the first segment (13);
the step of positioning the second ground layer (26) comprises a step of spreading a predetermined quantity of ground suitable for contacting at least partially the first and second segments (13; 14) of the reinforcing element (7).

In a 5th aspect according to the preceding aspect, the step of spreading the filling material to cover at least partially said reinforcing element (7), comprises at least the following sub-steps:
- spreading a portion of the predetermined quantity of material on the first segment (13) of the reinforcing element (7);
- compacting the portion of the spread material;
- repeating the steps of spreading and compacting until it is reached the height of the second segment (14) of the reinforcing element (7).

In a 6th aspect according to anyone of the preceding aspects, the step of providing the side wall (4) comprises, after the step of positioning the second ground layer (26), a step of turning at least a front portion (27) of the reinforcing element (7) over said second ground layer (26), the turning over step being suitable for placing the reinforcing element (7) in a three-dimensional configuration suitable for defining, according to a transversal view of the side wall (4), a substantially "C" profile having its concavity facing away from the inner face (4a) of the side wall (4).

In a 7th aspect according to the preceding aspect, the step of turning over the front portion (27) of the reinforcing element (7) is suitable for defining a third segment (15) placed above the second ground layer (26) which is interposed between said first and third segments (13; 15), the first and third segments (13; 15) being spaced and substantially parallel to each other, the second segment (14) connecting said first and third segments (13; 15) and being transversal to these latter, particularly said second segment (14) extending substantially parallel to the inner face (4a) of the side wall (4).

In an 8th aspect according to the preceding aspect, the first and third segments (13; 15) extend along respective rectilinear development directions, the development directions, under conditions of use of tank (1), being substantially horizontal.

In a 9th aspect according to anyone of the preceding aspects, the step of providing the ground layers comprises, before positioning the reinforcing elements (7), at least a further step of positioning a plurality of formworks (28) substantially above the upper abutment surface (34) and substantially at the rising surface (33), each formwork (27) being configured for supporting a front portion (27) of at least one reinforcing element (7) to define said second segment (14) .

In a 10th aspect according to the preceding aspect, the step of providing the ground layers comprises, after the positioning of the reinforcing element (7) and before spreading the filling material above the latter, at least a further step of positioning a plurality of formworks (28), each formwork (28) being interposed between at least one reinforcing element (7) and the compartment receiving the tank.

In an 11th aspect according to anyone of the aspects from 6 to 10, the step of turning over the front portion (27) of the reinforcing element (7) is suitable for positioning the third segment (15) at a minimum distance from the first segment (13) comprised between 0.5 m and 1.0 m, particularly between 0.5 m and 0.8 m.

In a 12th aspect according to anyone of the aspects from 6 to 11, the step of turning over the front portion (27) of the reinforcing element (7) is suitable for locating the third segment (15) at a minimum distance from the first segment (13) which substantially coincides with the height of a reinforced layer (9).

In a 13th aspect according to anyone of the preceding aspects, the step of positioning the reinforcing elements (7) comprises a sub-step of partially overlapping the reinforcing elements (7) of the same layer (9) so that these latter define, around the base (3), a closed outline.

In a 14th aspect according to anyone of the preceding aspects, the step of forming a plurality of reinforced layers (9) is suitable for defining a side wall (4) having an inner face (4a) extending transversally to the base (3) along all the outer perimeter of the latter.

In a 15th aspect according to the preceding aspect, the inner face (4a) of the side wall (4), along a cross section, extends along a substantially rectilinear prevalent development direction.

In a 16th aspect according to anyone of the preceding aspects, the step of providing the side wall (4) is configured for forming an inner face (4a) defining with the base (3) an obtuse angle.

In a 17th aspect according to the preceding aspect, wherein the angle defined between the base (3) and inner face (4a) is comprised between 95° and 130°, still more particularly between 100° and 120°.

In an 18th aspect according to anyone of the preceding aspects, the step of providing the side wall (4) is configured for forming a free edge (6) having an extension greater than the extension of an outer perimeter of base (3).

In a 19th aspect according to the preceding aspect, the ratio of the free edge (6) extension to the extension of the outer perimeter of the base is greater than 1, particularly greater than 1.1, still more particularly greater than 1.2.

In a 20th aspect according to anyone of the preceding aspects the step of forming the containing site (2) is configured for defining a base (3) having a development surface comprised between 150 m² and 2,000 m², particularly between 300 m² and 1,500 m², still more particularly between 500 m² and 1,300 m².

In a 21st aspect according to anyone of the preceding aspects, the step of forming the containing site (2) is configured for defining a free edge (6) extending substantially along a prevalent development plane, the containing site (2) having a height defined by the minimum distance between the prevalent development plane of the free edge (6) and the base (3), comprised between 5 m and 15 m, particularly between 6 m and 12 m, still more particularly between 6 m and 10 m.

In a 22nd aspect according to the preceding aspect, the height of the containing site (2) substantially coincides with the sum of the heights of the reinforced layers (9) forming the side wall (4).

In a 23rd aspect according to anyone of the preceding aspects, the step of providing the side wall (4) comprises a further step of forming an upper face (4b) connected to the free edge (6) of the side wall (4) transversally extending from the latter away from the base (3) along all the perimeter of the side wall (4) itself, the step of forming the upper face (4b) comprises at least the following sub-steps:
- positioning a predetermined quantity of ground on the last reinforcing layer (9);
- leveling the predetermined quantity of ground for defining on the top of the side wall (4), a flat abutment surface;
- compacting the predetermined leveled ground quantity.

In a 24th aspect according to the preceding aspect, the step of forming the upper face (4b) is configured for defining a flat surface having a width, defined by the minimum distance between the free edge (6) and an outer perimetral edge (11) of the upper face (4b), comprised between 1.5 m and 5 m, particularly between 2 m and 4 m, still more particularly between 2 m and 3 m.

In a 25th aspect according to anyone of the preceding aspects, the first and second elements (8a; 8b) of the reinforcing elements (7) are connected to each other at nodes in order to define meshes having a substantially rectangular or square shape.

In a 26th aspect according to anyone of the preceding aspects, the step of positioning the reinforcing elements (7) is suitable for positioning the first elements (8a) of each reinforcing element (7) substantially along a development direction parallel or tangent to the surface of the inner face (4a) at which said reinforcing element (7) is located.

In a 27th aspect according to anyone of the preceding aspects, the step of providing the reinforcing elements (7) comprises at least the following steps:
- co-extruding a series of first and second elements (8a; 8b) of plastics material to define a monolithic reticular structure of rectangular or square meshes;
- stretching the monolithic reticular structure along the development direction of second elements (8b) to obtain a mono-stretched reticular structure.

In a 28th aspect according to the preceding aspect, wherein, after the step of co-extruding the first and second elements (8a; 8b), said first elements (8a) are not stretched or have a stretch ratio less than the one of the second elements (8b), the stretch ratio of an element being defined as the ratio of the final length of the element itself once it has been stretched to the original length of such element before stretching it.

In a 29th aspect according to the aspects 27 or 28, the second elements (8b) have a stretch ratio greater than 3, optionally comprised between 4 and 10, more optionally between 5 and 8, the stretch ratio of the second elements being defined as a ratio of the final length of the second elements after having stretched the same to an original length of the second elements before being stretched.

In a 30th aspect according to the preceding aspect, the first elements (8a) during the turning over step, are kept parallel to each other and substantially horizontal.

In a 31st aspect according to anyone of the preceding aspects, the second elements (8b) are located in order to define, along a cross-section, an open outline shape and/or at least one segment oriented transversally to the surface of the inner face (4a).

In a 32nd aspect according to anyone of the preceding aspects, the step of forming the reinforcing elements (7) is configured for defining second elements (8b) having a length greater than 3 m, optionally greater than 5 m, still more optionally greater than 10 m, and first elements (8a) having a length greater than 0.5 m. In a 33rd aspect according to anyone of the preceding aspects, the step of forming the reinforcing elements (7) is configured for defining first and second elements (8a; 8b) having a solid cross-section.

In a 34th aspect according to anyone of the preceding aspects, the cross-section of the first elements (8a) being at least 5 times greater than the cross-section of the second elements (8b).

In a 35th aspect according to anyone of the preceding aspects, the first elements (8a) have a cross-section having an area greater than 20 mm², optionally greater than 30 mm².

In a 36th aspect according to anyone of the preceding aspects, the second elements (8b) have a cross-section having an area greater than 3 mm², optionally greater than 4 mm².

In a 37th aspect according to anyone of the preceding aspects, the step of forming the reinforcing elements (7) is configured for defining a reticular structure having an areal density (weight per unit surface) greater than 200 g/m², optionally between 200 and 1,200 g/m².

In a 38th aspect according to anyone of the preceding aspects, the step of forming the reinforcing elements (7) is configured for defining a reticular structure having a specific tensile strength, along the second elements (8b), greater than 20 kN/m, particularly comprised between 20 and 200 kN/m, optionally between 60 and 200 kN/m, said specific tensile strength being measured by the method set forth in the description.

In a 39th aspect according to anyone of the preceding aspects, the process comprises at least one further step of positioning a first covering layer (16) at least on the inner face (4c) of the side wall (4), said first covering layer (16) comprising at least one reticular structure net (19).

In a 40th aspect according to anyone of the preceding aspects, the process comprises at least one further step of positioning a second covering layer (17) at least on the inner face (4c) of the side wall (4) and on the base (3), said step of providing the second covering layer (17) being after the step of positioning the first covering layer (16).

In a 41st aspect according to the preceding aspect, the second covering layer (17) is positioned above the first covering layer (16) so that this latter is interposed between the inner face (4a) and second covering layer (17).

In a 42nd aspect according to anyone of the preceding aspects, the process comprises a further step of positioning a closing membrane (18) engaged with the containing site (2) and configured for hermetically closing the containing cavity (5).

In a 43rd aspect according to the preceding aspect, the step of positioning the closing membrane (18) is after the step of positioning the first and second covering layers (16; 17).

In a 44th aspect according to anyone of the preceding aspects, the process comprises, after the step of forming the side wall (4) of reinforced ground, a step of forming at least one trench (20) at the upper face (4b) of the side wall (4), said trench (20) having a housing seat (21) developing around the opening of the containing cavity (5) to define a closed outline, said housing seat (21) having an opening facing away from the base (3).

In a 45th aspect according to the preceding aspect, the step of forming the trench (20) is configured for defining a housing seat (21) having, along a cross-section, a substantially "C" profile having its concavity facing away from the base (3) of the containing site (2).

In a 46th aspect according to the aspects 44 or 45, the step of forming the trench (20) is configured for defining a housing seat (21) having, along a cross-section, a development surface comprised between 2,500 cm² and 10,000 cm², particularly between 3,000 cm² and 6,000 cm².

In a 47th aspect according to the aspects 44 or 45 or 46, the step of forming the trench (20) is configured for defining a housing seat (21) having a minimum height, defined by the minimum distance between a bottom surface (23) of the housing seat (21) and an edge (24) of the opening of the housing seat (21) itself, comprised between 50 cm and 100 m, particularly between 60 cm an 100 cm.

In a 48th aspect according to anyone of the aspects from 44 to 47, the process comprises a step of fixing the first covering layer (16), second covering layer (17) and closing membrane (18), said fixing step comprising at least the following steps:
- defining said trench (20) all around the opening of the containing cavity (5) to define a closed outline housing seat (21);
- positioning a perimetral end portion (30) of the first covering layer (16) inside the housing seat (21) of the trench (20);
- positioning a perimetral end portion (31) of the second covering layer (17) inside the housing seat (21) of the trench (20) on the first covering layer (16);
- positioning a perimetral end portion (32) of the closing membrane (18) inside the housing seat (21) of the trench (20) on the second covering layer (17);
- filling the housing seat (21) of trench (20) with a predetermined quantity of filling material after the preceding steps of positioning the covering layers (16; 17) and membrane (18).

In a 49th aspect according to the preceding aspect, the filling material comprises at least one of the following materials: ground, ballast, concrete, cement-stabilized sand.

In a 50th aspect it is provided a tank (1) for biogas producing plants comprising:
- at least one containing site (2) at least partially made of reinforced ground, comprising at least a base (3) and a side wall (4) of ground transversally emerging from base (3), said side wall (4) comprising at least one inner face (4a) which, with base (3), defines a containing cavity (5) arranged to receive a predetermined quantity of organic matter configured for forming a biogas following an anaerobic bacterial fermentation, the containing site (2) comprising a free edge (6) arranged away from the base (3) and defining an opening of the containing cavity (5);
- a plurality of reinforcing elements (7), each reinforcing element having a reticular structure (8), each reinforcing element (7) comprising:
   - a plurality of first elements (8a) spaced from each other, and having an elongated shape along a respective main extension direction,
   - a plurality of second elements (8b) spaced from each other, which substantially extend in a direction transversal to said first elements (8a), the second elements (8b) having also an elongated shape along a corresponding main extension direction;
said reinforcing elements (7) being arranged outside the containing cavity (5) inside the side wall (4) at the inner face (4a), at least some of said reinforcing elements (7) being connected to each other around the perimeter of the side wall (4) to define a continuous layer of reinforcing elements defining around the containing cavity (5) a closed outline, said continuous layer of reinforcing elements (7) with the ground of the side wall (4) defining a reinforced layer (9), said reinforcing elements (7) being configured for defining a plurality of reinforced layers (9) arranged on each other in order to define said inner face (4a).

In a 51st aspect according to the preceding aspect, the reticular structure (8) comprises a monolithic structure, at least partially flexible, of plastics material.

In a 52nd aspect according to anyone of the preceding aspects of tank, the base (3) substantially extends along a prevalent development plane, said side wall (4) being perimetrally connected to the base (3), particularly the base (3), under a condition of use of tank (1), substantially extends along a horizontal plane.

In a 53rd aspect according to anyone of the preceding aspects of tank, the inner face (4a) of the side wall (4), along a cross-section, extends along a substantially rectilinear prevalent development direction.

In a 54th aspect according to anyone of the preceding aspects of tank, the inner face (4a) defines with the base (3) an obtuse angle.

In a 55th aspect according to anyone of the preceding aspects of tank, the angle defined between the base (3) and inner face (4a) is comprised between 95° and 130°, still more particularly between 100° and 120°.

In a 56th aspect according to anyone of the preceding aspects of tank, the base (3) has a circular shape.

In a 57th aspect according to anyone of the preceding aspects of tank, the side wall (4) defines a frusto-conical shape.

In a 58th aspect according to anyone of the preceding aspects of tank, the base (3) has a development surface comprised between 150 m² and 2,000 m², particularly between 300 m² and 1,500 m², still more particularly between 500 m² and 1,300 m².

In a 59th aspect according to the aspects 56 or 57 or 58, the circular-shaped base (3) has a diameter comprised between 15 m and 50 m, particularly comprised between 20 m and 45 m, still more particularly between 25 m and 40 m.

In a 60th aspect according to anyone of the preceding aspects of tank, the free edge (6) of the side wall (4) extends along a prevalent development plane, said containing site (2) has a height, defined by the minimum distance between the prevalent development plane of the free edge (6) and the base (3), comprised between 5 m and 15 m, particularly between 6 m and 12 m, still more particularly between 6 m and 10 m.

In a 61st aspect according to anyone of the preceding aspects of tank, the side wall (4) comprises at least an upper face (4b) connected to the free edge (6) of the side wall (4) extending transversally to this latter away from the base (3) along all the perimeter of the side wall (4) itself.

In a 62nd aspect according to the preceding aspect, the upper face (4b) extends along a prevalent development plane, the prevalent development plane of the upper face (4b), under a condition of use of tank (1), being substantially horizontal.

In a 63rd aspect according to the aspects 61 or 62, the upper face (4b) extends all around the side wall (4) between an inner perimetral edge (10), coinciding with the free edge (6), and an outer perimetral edge (11), said upper face (4b) having a minimum width, defined by the minimum distance between the inner perimetral edge (10) and the outer perimetral edge (11), comprised between 1.5 m and 5 m, particularly between 2 m and 4 m, still more particularly between 2 m and 3 m.

In a 64th aspect according to the aspects 61 or 62 or 63, the side wall (4) comprises an outer face (4c) connected to the upper face (4b) and emerging transversally from the latter, said outer face (4c) emerging approaching the base (3) and away from the inner face (4a), the upper face (4b) being interposed between the inner face (4a) and outer face (4c).

In a 65th aspect according to the preceding aspect, the inner face (4c) extends from the outer perimetral edge (11) of the upper face (4b).

In a 66th aspect according to the aspects 64 or 65, the outer face (4c) of the side wall (4), along a cross-section, extends along a substantially rectilinear prevalent development direction.

In a 67th aspect according to the preceding aspect, the outer face (4c) defines with the upper face (4b) an inner obtuse angle.

In a 68th aspect according to the preceding aspect, the angle defined between the outer face (4c) and upper face (4b) is comprised between 95° and 150°, particularly between 95° and 130°, still more particularly between 100° and 120°.

In a 69th aspect according to the aspects 67 or 68, the angle comprised between the outer face (4c) and upper face (4b) is substantially equal to the angle defined between the base (3) and inner face (4a).

In a 70th aspect according to anyone of the aspects from 64 to 69, the outer face (4c) of the side wall (4) defines substantially a frusto-conical surface.

In a 71st aspect according to anyone of the aspects from 64 to 70, the outer face (4c) extends to an outer reference plane (12), the outer reference plane (12), under a condition of use of tank (1), is above or at the same level of base (3).

In a 72nd aspect according to the preceding aspect, the outer face (4c) vertically extends for the distance defined between the prevalent development plane of the free edge (6) and the outer reference plane (12), the outer face height being less than 16 m, particularly comprised between 4 m and 12 m, still more particularly between 6 m and 10 m.

In a 73rd aspect according to anyone of the preceding aspects of tank, the first and second elements (8a; 8b) of the reinforcing elements (7) are connected to each other at nodes in order to define meshes having a substantially rectangular or square shape, the first elements (8a) of each reinforcing element (7) substantially extending along a development direction parallel or tangent to the surface of the inner face (4a) at which said reinforcing element (7) is positioned.

In a 74th aspect according to the preceding aspect, the second elements (8b) of the reinforcing element (7), after their formation, are stretched along their prevalent development direction, the stretch ratio of an element being defined as the ratio of the final length of the element itself once it has been stretched to the original length of such element before stretching it.

In a 75th aspect according to the aspects 73 or 74, the first elements (8a) are not stretched or have a stretch ratio less than, optionally at least half, the one of the second elements (8b), the stretch ratio of an element being defined as a ratio of the final length of the same element once has been stretched, to the original length before being stretched.

In a 76th aspect according to the aspects 74 or 75, the second elements (8b) have a stretch ratio greater than 3, optionally comprised between 4 and 10, more optionally between 5 and 8, the stretch ratio of the second elements being defined as the ratio of the final length of the second elements after stretching the same to an original length of the second elements before stretching them.

In a 77th aspect according to anyone of the preceding aspects of tank, the second elements (8b) have a length greater than 3 m, optionally greater than 5 m, still more optionally greater than 10 m, and wherein the first elements (8a) have a length greater than 0.5 m.

In a 78th aspect according to anyone of the preceding aspects of tank, the first elements (8a) and second elements (8b) have a solid cross-section.

In a 79th aspect according to anyone of the preceding aspects of tank, the cross-section of first elements (8a) is at least 5 times greater than the cross-section of second elements (8b).

In an 80th aspect according to anyone of the preceding aspects of tank, the first elements (8a) have a cross-section area greater than 20 mm², optionally greater than 30 mm².

In an 81st aspect according to anyone of the preceding aspects of tank, the second elements (8b) have a cross-section area greater than 3 mm², optionally greater than 4 mm².

In an 82nd aspect according to anyone of the preceding aspects of tank, the reticular structure has an areal density (weight for unit surface) greater than 200 g/m², optionally between 200 and 1,200 g/m².

In an 83rd aspect according to anyone of the preceding aspects of tank, the reticular structure has a specific tensile strength, along the second elements (8b), greater than 20 kN/m, particularly comprised between 20 and 200 kN/m, optionally between 60 and 200 kN/m, said specific tensile strength being measured by the method set forth in the description.

In an 84th aspect according to anyone of the preceding aspects of tank, each reinforcing element (7) is positioned according to a three-dimensional configuration suitable for defining, along a transversal view of the side wall (4), a substantially "C" profile having a concavity facing away from the inner face (4a) of side wall (4).

In an 85th aspect according to the preceding aspect, the open outline reinforcing element (7) has at least one first length (13), one second length (14) and one third length (15), said first and third segments (13; 15) being spaced and substantially parallel to each other, said second segment (14) connecting said first and third segments (13; 15) and being transversal to these latter, particularly said segment (14) extending substantially parallelly to the inner face (4a) of the side wall (4).

In an 86th aspect according to anyone of the preceding aspects of tank, the first and third segments (13; 15) extend along respective rectilinear development directions, said development directions, under a condition of use of tank (1), being substantially horizontal.

In an 87th aspect according to anyone of the preceding aspects of tank, the second segment (14) extends along a substantially rectilinear prevalent development direction.

In an 88th aspect according to the aspects 85 or 86 or 87, each reinforcing element (7) has a height, defined by the minimum distance between the first and third segments (13; 15), comprised between 0.5 m and 1.0 m, particularly between 0.5 and 0.8 m.

In an 89th aspect according to anyone of the preceding aspects of tank, the height of each reinforcing element substantially coincides with the height of the reinforced layer (9).

In a 90th aspect according to anyone of the preceding aspects of tank, the tank comprises at least one first covering layer (16) suitable for covering at least the inner face (4a) of the side wall (4).

In a 91st aspect according to the preceding aspect, the first covering layer (16) comprises at least one reticular structure net (19).

In a 92nd aspect according to anyone of the preceding aspects of tank, the tank comprises at least one second covering layer (17) suitable for covering at least the inner face (4a) of the side wall (4) and base (3).

In a 93rd aspect according to the preceding aspect, the second covering layer (17) is positioned on the first covering layer (16) so that this latter is interposed between the inner face (4a) and second covering layer (17).

In a 94th aspect according to the aspects 92 or 93, the second covering layer comprises a membrane preferably made of a polymeric material, such as for example HDPE.

In a 95th aspect according to anyone of the preceding aspects of tank, the tank comprises a closing membrane (18) engaged with the side wall (4) of the containing site (2) and configured for closing the containing cavity (5) to define a closed volume tank.

In a 96th aspect according to the preceding aspect, the closing membrane (18) comprises a membrane preferably made of polymeric material, such as for example PVC.

In a 97th aspect according to anyone of the preceding aspects of tank, the tank comprises at least one trench (20) having a housing seat (21) developing around the opening of the containing cavity (5), to define a closed outline, said housing seat having an opening facing away from the base (3).

In a 98th aspect according to the preceding aspect, the housing seat (21) of the trench (20) has, along a cross-section, a substantially "C" profile, having the concavity facing away from the base (3) of the containing site (2).

In a 99th aspect according to the aspects 97 or 98, at least one of the first covering layer (16), second covering layer (17) and closing membrane (18) is at least partially positioned inside the housing seat (21) of trench (20), said tank (1) further comprising a predetermined quantity of filling material (22) located inside the housing seat (21) on a portion of at least one of the first covering layer (16), second covering layer (17) and closing membrane (18).

In a 100th aspect according to the preceding aspect, the filling material comprises at least one of the following materials: ground, ballast, concrete, cement-stabilized sand.

In a 101st aspect according to anyone of the aspects from 97 to 100, the housing seat (21), along a cross-section, comprises a development surface comprised between 2,500 cm² and 10,000 cm², particularly between 3,000 cm² and 6,000 cm².

In a 102nd aspect according to anyone of the aspects from 97 to 101, the housing seat (21) of the trench (20) has a minimum height, defined by the minimum distance between a bottom surface (23) of the housing seat (21) and an edge (24) of the opening of the housing seat (21) itself, comprised between 50 cm and 100 cm, particularly between 60 cm and 100 cm.

In a 103rd aspect according to anyone of the aspects from 97 to 102, the trench (20) is positioned on the upper face (4b) of side wall (4).

In a 104th aspect according to anyone of the aspects from 99 to 103, the first covering layer (16), the second covering layer (17) and the closing membrane (18) have a respective perimetral end portion positioned inside the housing seat (21) along all over the closed outline, the filling material (22) being positioned on said end portions and being configured for stably blocking these latter.

In a 105th aspect according to anyone of the preceding aspects of tank, wherein the tank comprises a plurality of formworks (28) configured for supporting a front portion (27) of at least one reinforcing element (7).

In a 106th aspect according to the preceding aspect, each formwork (28) is interposed between at least one reinforcing element (7) and the housing compartment of the tank.

In a 107th aspect according to the aspects 105 or 106, each formwork (28) comprises a supporting frame, particularly made of metal material, defining, along a cross-section, a substantially "L" profile, the "L" frame of each formwork (28) being at least partially countershaped to the reinforcing element which is associated to.

In a 108th aspect according to anyone of the aspects from 105 to 107, each formwork (28) comprises a first supporting segment positioned at the first segment of the reinforcing element which is associated to, each formwork (28) further comprises a second supporting segment positioned at the second segment of the reinforcing element which is associated to.

In a 109th aspect according to anyone of the aspects from 9 to 49, each formwork (28) is interposed between at least one reinforcing element (7) and the housing compartment of tank.

In a 110th aspect according to anyone of the aspects from 9 to 49 or 109, each formwork (28) comprises a supporting frame, particularly made of metal material, defining, along a cross-section, a substantially "L" profile, the "L" frame of each formwork (28) being at least partially countershaped to the reinforcing element which is associated to.

In a 111th aspect according to anyone of the aspects from 9 to 49 or 109 or 110, each formwork (28) comprises a first supporting segment positioned at the first segment of the reinforcing element which is associated to, each formwork (28) further comprising a second supporting segment positioned at the second segment of the reinforcing element which is associated to.

In a 112th aspect it is provided a biogas producing plant (100) comprising:
- at least one first circuit (101) developing between an inlet (101a) and an outlet (101b), said first circuit (101) being configured for enabling an organic matter to circulate between said inlet (101a) to said outlet (101b);
- at least one tank (1) according to anyone of the preceding aspects of tank, said tank (1) being fluidically communicating with the first circuit (101) and being configured for enabling to digest and/or store a predetermined quantity of the organic matter, said tank (1) being configured for generating a biogas following an anaerobic bacterial fermentation of the organic matter;
- at least one second circuit (102) fluidically communicating with said tank (1) and configured for enabling the circulation of biogas generated by said tank.

In a 113th aspect according to the preceding aspect, the plant comprises:
- at least one digestion tank (1) configured for enabling to digest the organic matter for generating biogas;
- at least one storage tank (1) positioned between the digestion tank (1) and the outlet (101b) of the first circuit (101), said storage tank (1) being configured for receiving the processed organic matter exiting the digestion tank (1).

In a 114th aspect according to the preceding aspect, the second circuit (102) is fluidically communicating with said digestion and storage tanks (1).

### BRIEF DESCRIPTION OF THE DRAWINGS

Some embodiments and some aspects of the invention will be described in the following with reference to the attached drawings, provided in an indicative and therefore non limiting way wherein:
- Figure 1 is a schematic view of a plant for producing biogas according to the present invention;
- Figures 2, 3, and 4 are cross-section views of different embodiments of a tank for biogas producing plants;
- Figure 5 is a detailed view of the cross-section of Figure 4;
- Figure 6 is a top view of a tank for biogas producing plants according to the present invention;

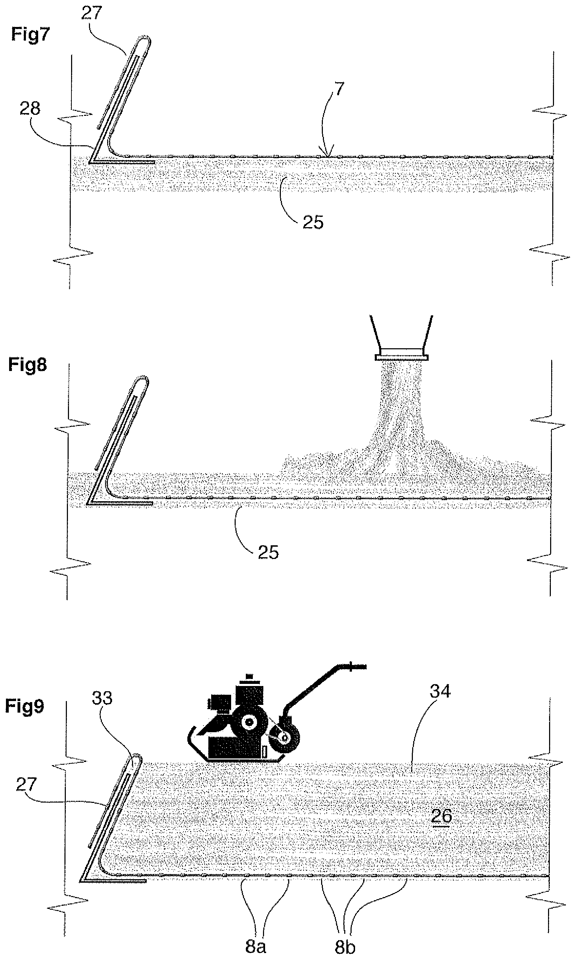

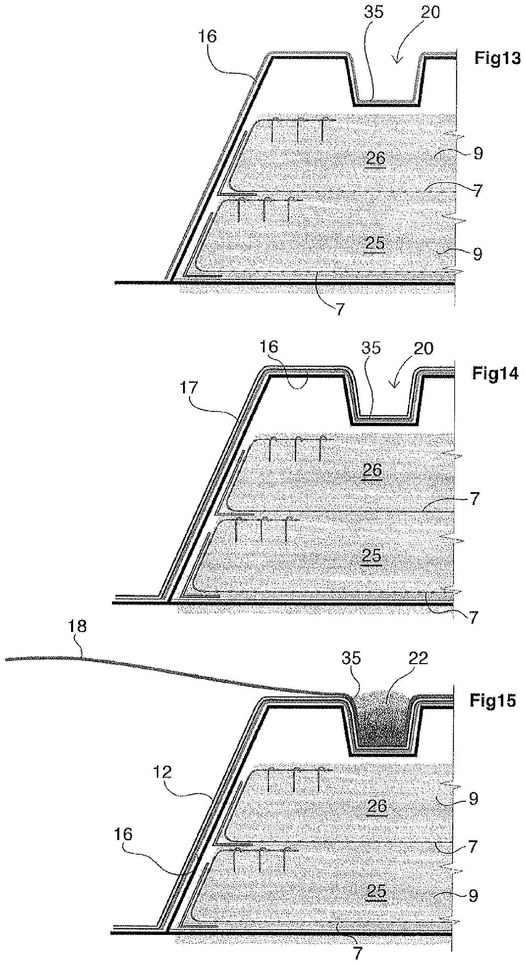

- Figures 7 - 15 schematically show the steps of a process for forming a tank for biogas producing plants according to the present invention;
- Figure 16 is a top view of a portion of the reinforcing element according to the present invention.

### DETAILED DESCRIPTION

### Tank for biogas producing plants

1 generally indicates a tank for plants 100 for producing biogas obtained by an anaerobic bacterial fermentation (without oxygen) of organic residuals coming from: waste, decomposing plants, zootechnical waste or depuration sludge, agro-industry waste. Tank 1, object of the present invention, can be used for defining plants of any "size", in other words for small, medium and large biogas producing plants 100. Tank 1 can be for example used in small-sized plants having a power output of about 100 KWe, and in large-size plants having a power output greater than 1,000 KWe.

The whole process consists of decomposing an organic matter by some types of bacteria, in order to produce carbon dioxide, molecular hydrogen and methane (methanation of organic compounds).

The biogas can be used for being combusted in heating boilers, for producing electric energy or, if it suitably depurated (separated from other components such as carbon dioxide and sulphur), can be used as automotive bio-methane (methane cars and vehicles).

Generally, the biogas producing plants 100 consist of one or more anaerobic digesters comprising substantially tightly-closed and insulated reservoirs suitable for receiving the organic matter: the fermentation process takes place inside the digester, which due to anaerobic microorganisms present inside the reservoir itself, enables to obtain biogas and/or (solid and liquid) bio-fertilizers.

Further, the biogas plants 100 can comprise one or more storage reservoirs configured for receiving the processed organic matter exiting the digesters. The present discussion will refer to a tank 1 for plants 100, which can be used both for implementing digesters (reservoir for digesting organic matter) and for implementing storage reservoirs.

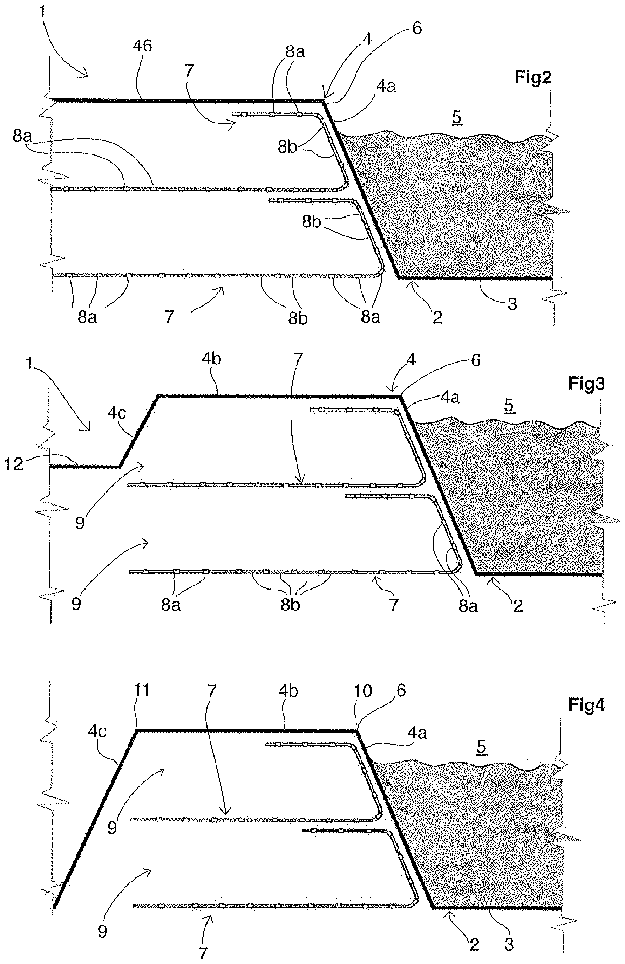

As it is visible for example in Figures 2-4, tank 1 comprises a containing site 2 made at least partially with reinforced ground: the containing site comprises a base 3 and a side wall 4 of ground transversally emerging from base 3.

Base 3 substantially defines an abutment surface of organic matter, preferably extending along a prevalent development plane; the prevalent development plane of base 3, under conditions of use of tank 1, is substantially horizontal.

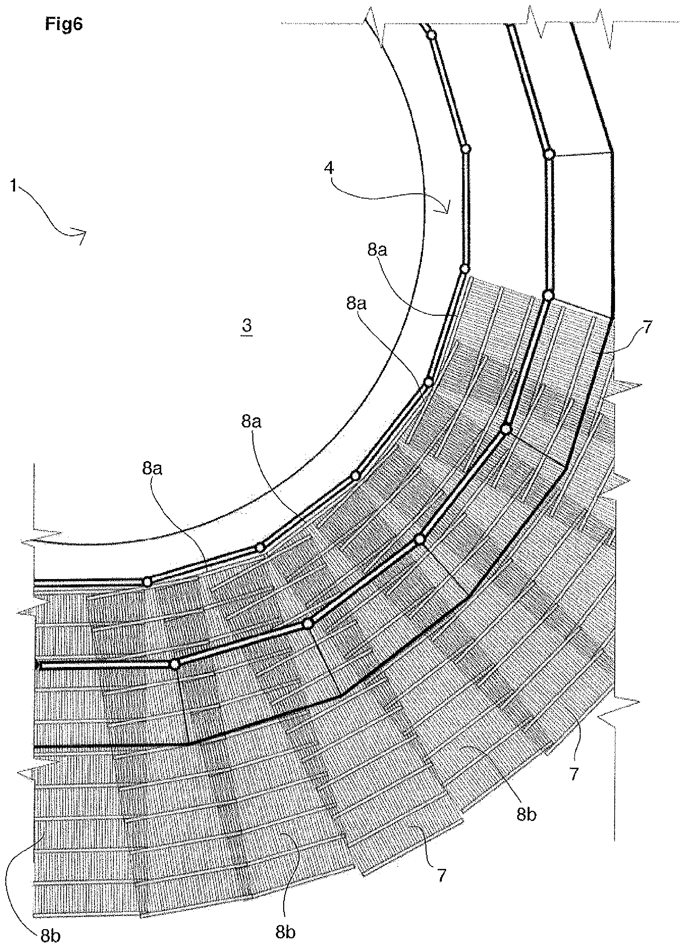

According to the geometrical outline, base 3 can define different outlines, such as for example a circular, rectangular, or square outline. The detailed view of Figure 6 schematically shows, in a non limiting way, a tank 1 comprising a base 3 having a substantially circular shape.

According to the dimensional outline, base 3 has a development surface between 150 m² and 2,000 m², particularly between 300 m² and 1,500 m², still more particularly between 500 m² and 1,300 m². When the base 3 has a circular shape, the diameter defined by the latter is comprised between 15 m and 50 m, particularly comprised between 20 m and 45 m, still more particularly between 25 m and 40 m.

Advantageously, base 4 is made of ground particularly obtained by excavations and then by leveling the ground. However, further embodiments of base 3 are not excluded, for example it can be made of cement.

As it is visible in the attached figures, the side wall 4 of reinforced ground extends from the outer perimeter of base 3. The side wall 4 emerges away from base 3 and with this latter defines a containing cavity 5 adapted to receive a predetermined quantity of organic matter configured for generating a biogas following an anaerobic bacteria fermentation.

As it is visible from the attached figures, the side wall 4 has an inner face 4a facing the base 3; particularly it is the inner face 4a defining the containing cavity 5 with base 3. The side wall 4 face 4a extends all around base 3 in order to define a closed outline. Preferably, the inner face 4a of the side wall 4, along a cross-section, extends along a substantially rectilinear prevalent development direction. The inner face 4a defines with base 3 an obtuse angle; particularly, the angle defined between the base 3 and inner face 4a is comprised between 95° and 130°, still more particularly between 100° and 120°. De facto, the inner face 4a represents a surface warped in the direction of base 3.

As it is visible in the attached figures, inner face 4a has a free edge 6 delimiting an upper opening of cavity 5 of the containing site 2. De facto, the free edge 6 represents a perimetral edge of the inner face 4a positioned opposite the base 3 with respect to the side wall 4 itself.

In a preferred embodiment of tank 1, the free edge 6, defined by inner face 4a, has the same shape as the outer perimetral edge of base 3. De facto, the base in the preferred embodiment shown in Figure 6, has a substantially circular shaped outer perimetral edge; under such condition also the free edge 6 of the inner face 4a has a circular shape to define an inner frusto-conical face 4a. However, workable variants of face 4a wherein this latter has a free edge 6 having a shape different than the profile of the outer perimeter of base 3 (configuration not shown in the attached figures) are not excluded. The free edge 6 of face 4a extends, in a non limiting way, along a prevalent development plane which, under conditions of use of tank 1, is substantially horizontal; the distance between the development plane of free edge 6 and base 3 defines the height of the containing site 2 which is comprised between 5 m and 15 m, particularly between 6 m and 12 m, still more particularly between 6 m and 10 m.

The volume of the containing cavity 5 is comprised between 1,000 m³ and 47,000 m³, particularly between 2,000 m³ and 26,000 m³, still more particularly between 3,000 m³ and 17,000 m³.

As it is visible in the attached figures, side wall 4 can further comprise an upper face 4b connected to the free edge 6 of side face 4 extending transversally to this latter away from base 3 along all the perimeter of the side wall 4 itself. More particularly, the upper face 4b extends along a prevalent development plane which, under a condition of use of tank 1, is substantially horizontal. As it is visible for example in Figures 3 and 4, the upper face 4b extends parallelly to base 3 to substantially define an upper abutment plane.

As for the inner face 4a, the upper face 4b extends all around the side wall 4 between the inner perimetral edge 10, coinciding with the edge 6, and a perimeter outer edge 11 (Figure 4).

According to the dimensional outline, the upper face 4b has a minimum width defined by the minimum distance between the inner perimetral edge 10 and the outer perimetral edge 11, comprised between 1,5 m and 5 m, particularly between 2 m and 4 m, still more particularly between 2 m and 3 m.

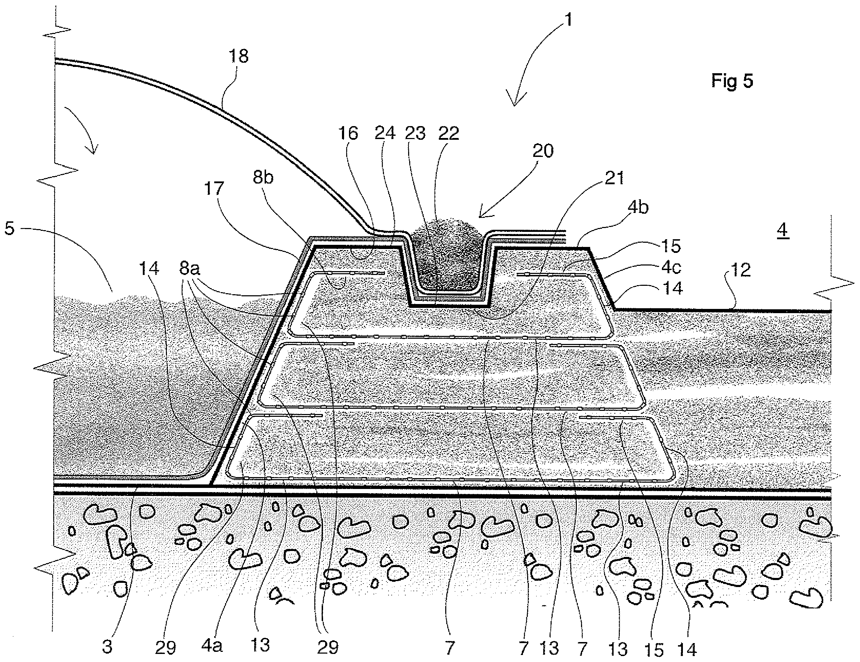

In a further embodiment of tank 1 shown in Figure 5, side wall 4 comprises an outer face 4c connected to the upper face 4b and emerging transversally from this latter; the outer face 4c emerges approaching the base 3 and away from the inner face 4a. De facto, in the configuration of the side wall 4 shown in Figure 5, upper face 4b is substantially interposed between the inner face 4a and outer face 4c.

As for the inner face 4a and upper face 4b, also outer face 4c extends all around the base 3 to define, around the containing cavity 5, a closed outline.

According to the geometrical outline, the outer face 4c of side wall 4, along a cross-section, extends along a substantially rectilinear prevalent development direction which defines with the upper face 4b an inner obtuse angle; the angle defined between the outer face 4c and upper face 4b is comprised between 95° and 150°, particularly between 95° and 130°, still more particularly between 100° and 120°. The outer face 4c defines a frusto-conical shape inverted with respect to the frusto-conical shape defined by the inner face 4a.

In a preferred, but not limiting embodiment of the invention, the obtuse angle comprised between outer face 4c and upper face 4b is substantially equal to the obtuse angle defined between base 3 and inner face 4a.

As it is visible in Figure 3, the outer face 4c extends from the upper face 4b to an outer reference plane 12 which, under a condition of use of tank 1, is substantially horizontal.

Figure 3 shows a preferred, but not limiting, embodiment of tank 1 in which the outer reference plane 12 of outer face 4c is at a height greater than the height of base 3: under such condition the vertical extension of outer face 4c is less than the vertical extension of inner face 4a.

Alternatively, the reference plane 12 of outer face 4c can be substantially at the same height as base 3 (a condition not shown in the attached figures).

According to the dimensional outline, the outer face 4c vertically extends for the distance defined between the prevalent development plane of the free edge 6 and the outer reference plane 12: the height of the outer face 4c is less than 16 m, particularly is comprised between 4 m and 12 m, still more particularly between 6 m and 10 m.

As previously described, the side wall 4 is a containing structure made of reinforced ground; more particularly, the side wall 4 of tank 1 comprises inside a plurality of reinforcing elements 7 having an at least partially flexible reticular structure 8. The reinforcing elements 7 are substantially formed by nets configured for reinforcing/compacting the side wall 4 so that the inner face 4a can resist to the force exerted by the organic matter received inside the containing cavity 5.

De facto, the reinforcing element 7 comprises a reticular structure or grid 8 which, in a preferred but not limiting configuration of the invention, comprises an integral structure of plastics material: a monolithic structure integrally obtained and not formed by glued connections or by weaving different elongated elements. The reticular structure comprises a series of first elements or bars, 8a, spaced and parallel to each other; the first elements or bars 8a are interconnected by a plurality of second elements 8b, which are also for example spaced and parallel to each other. In the present discussion, it is understood that each of the first elements 8a extends for all the width of the reticular structure 8, in other words it is formed by a plurality of portions aligned along the same line of the reticular structure 8. Similarly, each of the second elements 8b extends in the direction of the length of the reticular structure 8, in other words it is formed by the plurality of portions aligned along the same line of the reticular structure 8: in such way each first elements 8a are intersected by a plurality of second elements 8b and each second elements 8b are intersected by a plurality of first elements 8a.

In a preferred, but non limiting embodiment of the invention, the reticular structure 8 is stretched along the development of second elements 8b (the second elements 8b of reinforcing element 7 are stretched along the prevalent development direction) to define a mono-stretched net or grid. In the preferred described and illustrated embodiment, the monolithic reticular structure 8 of plastics material is stretched, in a non limiting way, only along the direction of second elements 8b; however the use of reticular structures stretched along two different prevalent development directions (bi-stretched nets) is not excluded.

According to the stretch degree, second elements 8b have a structure which is more or less thin, which can also have a threadlike shape; anyway, second elements 8b have a cross-section which at an intermediate point between two consecutive bars 8a is substantially smaller than the cross-section of bars 8a.

More particularly, first elements or bars 8a have a substantially not stretched structure (or at least slightly stretched) and a thickness (and a cross-section) constantly greater than the thickness (and the cross-section) of the second elements or threads 8b. So, first elements or bars 8a have a greater compressive strength and show a greater friction with the ground than second elements 8b.

Second elements 8b, for example having a shape of longitudinal threads, are connected to the bars 8a and have a substantially stretched structure along their development such to give them a greater tensile strength per unit area than bars 8a.

The reticular structure 8, useable for forming the reinforcing element 7, can be a preform obtained by an extruded (or calendered or laminated or molded) plate provided with or devoid of through holes, or a preform obtained by extrusion and then drilled by precursors of first elements 8a and simultaneously forming the precursors of the second elements 8b positioned transversally to the precursors of first elements 8a.

In the first case, a constant thickness preform will be obtained except for the drilled areas, while in the second case a manufacture having a variable thickness and outline will be obtained. By longitudinally stretching the precursors of the second elements 8b it is obtained the reticular structure 8 of the reinforcing elements 7.

According to whether it is used one or the other of the above described preforms, the distance between adjacent bars or first elements 8a can be varied by modulating the beat rates during the plate drilling step or the pitch by which the precursors of the second elements 8b are applied during the extrusion step, and the longitudinal stretch ratios. The distance between adjacent second elements 8b can be varied by modifying, according to whether it is used one or the other of the above described preforms, the tooling of the plate drilling step or of the step of forming the precursors of second elements 8b. This enables to obtain a greater or smaller number of first and/or second elements 8a, 8b and to vary their thickness, as a function of the longitudinal stretch applied to second elements 8b and the pitch between first elements 8a. The application of equal but opposed stretch forces causes the bars 8a to be uniformly spaced and parallel and aligned, devoid of bends or slants which could compromise their correct use.

As previously described, in the preferred embodiment of the invention, the reticular structure 8, and particularly the elements 8a and 8b, can be made of plastics material, selected based on the physical and mechanical characteristics of the material itself; for example elements 8a and 8b can be made of HDPE or polypropylene or also of other polymers.

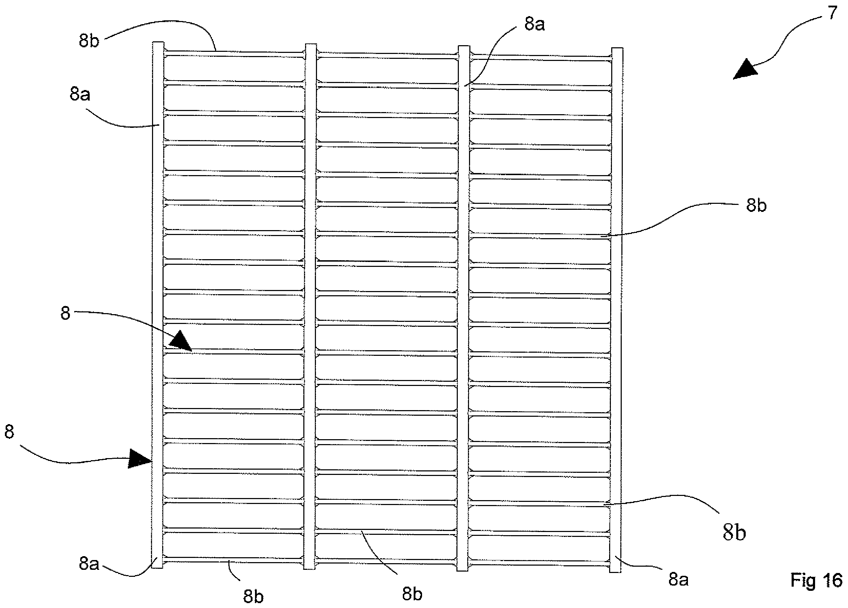

As it is visible in Figure 16, first and second elements 8a, 8b intersect at nodes in order to form meshes having a rectangular or square shape. Each first and second element 8a, 8b have portions extending between consecutive nodes. Due to the longitudinal stretch, the portions of second elements 8b have terminal areas having a cross-section (measured orthogonal to the main development direction of second elements) progressively decreasing from a node in the direction of a centerline of the portions and a central area having a substantially constant cross-section (a configuration not shown in the attached figures).

According to the dimensional outline, the reticular structure 8 has a width defined by the distance between two longitudinal edges of the structure 8 itself (the width coincides with the extension of first elements 8a); such width is greater than 0.5 m, particularly is comprised between 1.0 m and 2.0 m.

Whereas, with reference to the length of the reticular structure 8, this is substantially defined by the distance between two transversal edges of the net (coinciding with the length of second elements 8b); the length of the reticular structure is greater than 5 m, particularly is comprised between 12 and 30 m, still more particularly comprised between 15 and 25 m.

The length and width of the reticular structure 8 can depend on the manufacturing process used. De facto, whether it is used a reinforcing structure 7 obtained from a drilled plate or from a co-extruded element, the dimensions are dictated by the width capacity of the machine adapted to produce the reticular structure 8 and by the direction which the reticular structure 8 is stretched along. More particularly, if it is desired to obtain a mono-stretched net along an advancement direction of the reticular structure 8 on the machine (the second stretched elements 8b are parallel to the advancement direction of the reticular structure 8), such structure will have a reduced size along the first elements 8a and substantially coinciding with the width capacity of the machine, whereas along the advancement direction it will be possible to obtain extremely extended stretched second elements 8b because it is not present any manufacturing limits of the machine: possibly in such configuration the second elements 8b can have an unlimited extension.

Viceversa, it there is the need to form a reticular structure 8 mono-stretched along a direction transversal to the advancement direction on the machine for the same structure (second elements 8b extend transversally to the net advancement direction), such net will have an extension limited along the stretched elements: the limitation is dictated by the reduced width capacity of the machine transversally to the advancement direction and by the amount which the net is stretched by. Along the advancement direction, it is possible to obtain extremely extended first elements 8a because there is no manufacturing limit of the machine: possibly, in such a configuration, the first elements 8 can have an unlimited extension.

More particularly, the distance between two first adjacent elements 8a is comprised between 100 mm and 400 mm, optionally between 200 mm and 300 mm. Similarly, the distance between second adjacent elements 8b is comprised between 10 mm and 50 mm, optionally between 15 mm and 40 mm. Varying such distance varies the size of the meshes which can have a through area comprised between 1,000 and 20,000 mm². The figures show that the second elements 8b are positioned transversally, for example orthogonally, to the first elements 8a.

More specifically, it is possible to define the dimensions of the elements forming the reticular structure 8. As it is possible to note from the attached figures, first elements 8a are substantially unstretched (or at most slightly stretched, but always less than the amount which the second elements have been stretched by) and have a dimension and thickness substantially greater than the ones of the second elements 8b, this choice comes from the fact that the reinforcing element 7 must have a determined stiffness and a good anchoring capacity. On the contrary, since second elements 8b are markedly stretched and have a reduced cross-section, they are flexible and have an excellent tensile strength. De facto, first elements 8a, along a cross-section transversal to their prevalent development direction, have an area greater than 20 mm², optionally greater than 30 mm². Second elements 8b, along a cross-section transversal to their prevalent development direction, have an area greater than 3 mm², optionally greater than 4 mm². The second elements 8b are thinned down by the stretching process by which the reduction of the cross-section area and the longitudinal increase of second elements 8b are obtained. The stretch ratio, in other words the ratio of the length of second elements 8b after they have been stretched to the length of the second elements 8b before being stretched, is at least 3, optionally comprised between 4 and 10, more optionally between 5 and 8. It is noted that it is possible to give a slight stretch to first elements 8a (for example a stretch entailing a stretch ratio not greater than 1.5), provided that first elements 8a maintain the above described size in terms of longitudinal extension and cross-section area.

It is possible to identify the stretch of the elements because the process is capable of arranging the molecular chains forming second elements 8b according to an alignment more oriented along the prevalent development direction of the second elements themselves. As said before, the stretch process is executed along the second elements 8b development after having formed the reticular structure preform. The stretch process gives a greater tensile strength and flexibility to second elements 8b.

The obtained reinforcing element 7, which as said is preferably made of a plastics material, shows an areal density (weight per unit surface) from 200 to 1,200 g/m² and a specific tensile strength, along second elements, greater than 20 kN/m, particularly comprised between 20 and 200 kN/m, optionally between 60 and 200 kN/m. The tensile specific strength is measured according to the method set forth in the EN ISO 10319 standard.

For the sake of completeness, it is observed that, independently from a possible stretch, also first elements 8a can be also subjected to a slight warpage made by their passage through driving rolls and the cohesion to second elements 8b, particularly at and in proximity with the reticular structure 8 nodes.

The examples illustrated in figures show only a reticular structure 8 mono-stretched along second elements 8b. While the reticular structure 8 has two prevalent development directions (in other words the directions which the first and second elements extend along), structure 8 has also a determined size, or thickness, orthogonally to such prevalent development directions, which give to the reinforcing element 7 a three-dimensional structure markedly distinct from sheet materials. Particularly, the maximum thickness of the reticular structure 8 is greater than 3, for example 4 or 5 mm. The thickness is defined by the maximum distance between opposite sides of the reticular structure 8.

Since the second elements 8b have been subjected to the stretch process they have an average thickness, measured for example at an intermediate area between two consecutive nodes, much smaller than the thickness of first elements 8a.

The elongated shape of second elements 8b gives an optimal flexural capacity, particularly enables the reticular structure 8 of freely flexing along at least one transversal axis substantially parallel to first elements 8a.

As it is visible in the attached figures, tank 1 comprises a plurality of reinforcing elements 7, positioned outside the containing cavity 5 and inside the ground side wall 4 to define a side wall 4 of reinforced ground. Some reinforcing elements 7 are connected to each other around all the perimeter of the side wall 4: with the ground of the side wall 4, said reinforcing elements 7 connected to each other define a reinforced layer 9 horizontally positioned at the inner face 4a of side wall 4 to define a closed outline of the containing cavity 5.

Preferably, tank 1 comprises a plurality of reinforced layers 9 positioned on each other to define said inner face 4a (a preferred configuration illustrated in attached figures).

The reticular structure 8 of reinforcing element 7 is formed by a layer having a rectangular shape formed by first and second elements 8a, 8b (particularly the reticular structure 8 is only formed by the first and second elements 8a, 8b): the rectangular layer shape is dictated by the manufacturing process used.

In case the side wall 4 of tank 1 has a circular shape, in order to correctly reinforce the side wall 4 ground, each layer 9 has consecutively adjacent reinforcing elements 7 which are partially overlapped and constrained to each other (configuration shown in Figure 6). The partial overlap of reinforcing elements 7 is done at the inner face 4a: so that it is possible to define around the inner face 4a a disk formed by reticular structures 8 perimetrally closed around the containing cavity 5. Based on the overlapping degree of reinforcing elements 7, the reticular structures 8 disk will have a determined width defined by the distance between a hypothetical inner perimeter (the inner perimeter of the disk is positioned at the inner face 4a and is substantially countershaped to this latter) and a hypothetical outer perimeter.

In case a reinforced side wall 4 having a rectangular or squared shape (having rectilinear perimetral sides) should be defined, it is possible to reinforce the wall by connecting consecutively adjacent reinforcing elements 7 by longitudinal edges without overlapping the reinforcing elements 7.

Advantageously, each reinforcing element 7 is positioned inside the lateral wall 4 so that first elements 8a extend substantially along a development direction parallel or tangent to the inner face 4a surface at which said reinforcing element 7 is positioned. More particularly, if the side wall 4 has a circular development, the first elements 8a of a reinforcing element 7 will be substantially tangent to the inner face 4a portion at which said reinforcing element 7 is positioned.

As previously briefly described, the first elements 8a of the preferred embodiment are substantially not stretched or have a stretch ratio less than the one of second elements 8b; such condition enables to give a determined stiffness and determined anchoring capacity to the ground in the thrust direction of the organic matter on inner face 4a, to the reticular structure 8.

In such a condition, second elements 8b positioned transversally, particularly orthogonally to first elements 8a, are adapted to be subjected to a tensile strength opposite to the thrust of the ground in order to enable to consolidate the latter.

As it is visible in the attached figures, each reinforcing element 7 is positioned according to a three-dimensional configuration adapted to define, along a cross-section of side wall 4, a substantially "C" profile having a concavity facing away from the inner face 4a. Particularly, each reinforcing element 7 is suitable for defining, according to the side wall 4 cross-section, an open outline having at least one first, one second and one third segments (segments 13, 14 and 15, respectively). Each of said segments extends, in a non limiting way, along respective rectilinear development directions.

As it is visible for example in the cross-section view of Figure 5, the first and third segments 13, 15 substantially extend along a direction parallel to the development plane of base 3 and transversal to the inner face 4a: such segments (the first and third segments) are spaced and substantially parallel to each other.

Second segment 14 substantially defines a connecting (linking) element between the first and third segments 13, 15. As it is visible in the attached figures, second segment 14 substantially extends parallelly to the inner face 4a of side wall 4.

The distance between the first and third segments 13, 15 defines substantially the vertical extension of each reinforcing elements 7 and particularly the height of the reinforced layer 9; such height is substantially comprised between 0.5 m and 1.0 m, particularly between 0.5 m and 0.8 m. The sum of the reinforced layers 9 heights substantially defines the height (the vertical development) of containing site 2.

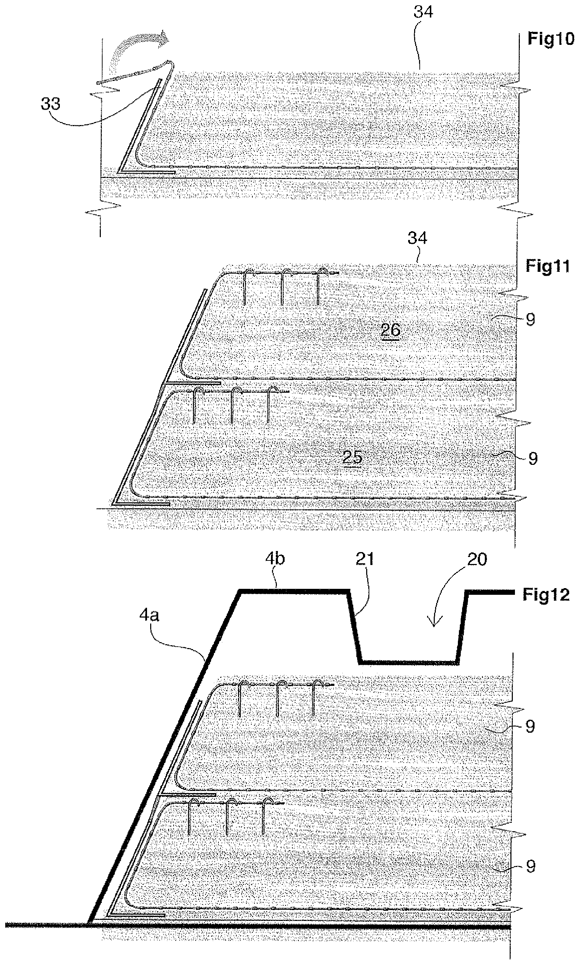

Further, as it is visible in the attached figures, tank 1 can comprise a predetermined number of formworks 28, each of them is substantially positioned at the inner face 4a. Formworks 28 are configured for defining supporting elements for reinforcing elements 7; each formwork 28 comprises substantially a supporting frame or rest having, along a cross-section, a substantially "L" profile: formworks are countershaped to the reinforcing element 7 which are associated to, particularly to first and second segments 13, 14 of reinforcing element 7 which are associated to. Under a condition of use of tank 1, the horizontal segment of the formwork 28 is positioned below and at first segment 13 of a reinforcing element 7 while the vertical segment of formwork 28 is positioned at second segment 14 of the reinforcing element 7 itself. In an embodiment of tank 1 (Figures 11 and 12), formworks 28 of a reinforced layer 9 are engaged to formworks 28 positioned on the immediately above reinforced layer 9.

Formworks 28, besides defining reinforcing/consolidating elements of inner face 4a, are suitable for correctly positioning the reinforcing element 7 during the tank 1 forming steps. The steps of positioning reinforcing elements 7 will be better described in the following.

As previously said, containing cavity 5 is adapted to receive and hold a predetermined quantity of organic matter; the thrust of the organic matter against the site 2 containing walls (base and side wall) is particularly high. For improving the compaction degree of the ground and leveling the inner face 4a surface, tank 1 can further comprise at least one first covering layer 16 configured for covering at least the inner face 4a, particularly on all the inner surface. First covering layer 16 can comprise a reticular structure net of plastics material and/of a polyurethane layer. Therefore it is possible to provide a covering element 16 comprising just a monolithic reticular structure or just a polyurethane covering layer; in an implementable variant, the covering layer comprises both the reticular structure and the polyurethane layer. First covering layer 16 has an average thickness comprised between 3 cm and 20 cm, particularly between 5 cm and 15 cm, still more particularly about 10 cm. Covering layer 16 is configured for covering possible projections (for example portions projecting from formworks 28) of inner face 4a and defining a substantially smooth surface.

Further, tank 1 can comprise at least one second covering layer 17 configured for covering at least the inner face 4a of side wall 4 and/or base 3; second covering layer 17 is positioned on first covering layer 16 so that this latter is interposed between the inner face 4a and second covering layer 17. Second covering layer 17 is not just a consolidating element but is a covering and finishing element for tank 1, configured for preventing the organic matter from leaking into the ground. The first covering layer 16 is configured for configuring the abutment of the second covering layer 17 on a surface devoid of projections which could cut for example the cover (second layer 17). De facto, second covering element 17 comprises a membrane preferably made of a polymeric material, such as for example HDPE.

As it is particularly visible for example in Figure 5, further tank 1 comprises at least one closing membrane 18 engaged with the side wall 4 of containing site 2 and configured for hermetically closing the containing cavity 5 to define a closed volume tank. Membrane 18 is substantially configured for containing and collecting inside the site 2 the biogas generated by the organic matter; de facto, biogas exiting the organic matter is hold between this latter and membrane 18. Advantageously, the membrane is made of a polymeric material, such as for example PVC.

Further, in a preferred but non limiting embodiment of the invention, illustrated for example in Figure 5, tank 1 comprises a trench 20 positioned at the upper face 4b of side wall 4; trench 20 comprises a housing seat 21 continuously developing on the upper face to define around the containing cavity 5 opening a closed outline. As it is particularly visible in Figure 5, housing seat has an opening facing away from the base 3. De facto, housing seat 21 of trench 20 has, along a cross-section transversal to side wall, a substantially "C" profile having a concavity facing away from base 3.

Housing seat 21, along a cross-section, comprises a development surface comprised between 2500 cm² and 10000 cm², particularly between 3000 cm² and 6000 cm². Particularly, seat 21 has a minimum height defined by the minimum distance between a bottom surface 23 of housing seat 21 and an edge 24 of the opening of the containing seat 21 itself, comprised between 0.5 m and 1.0 m, particularly about 0.60 m. De facto, the seat height is defined by the distance between the bottom surface 23 of seat 21 and the prevalent development plane of upper face 4b.

As previously described, the organic matter thrust inside the site 2 is particularly high; for such reason trench 20 is formed at a certain distance from inner face 4a in order to ensure the solidity of side wall 4. The proximity of trench 20 to inner face 4a could determine a weakening of side wall 4. De facto, the minimum distance between the free edge 6 of inner face 4a and an upper perimetral edge of housing seat is comprised between 0.5 m and 1 m, particularly between 0.6 and 0.75 m.

Housing seat 21 of trench 20 is configured for receiving at least one of the first covering layer 17, second covering layer 17 and closing membrane 18. In a preferred but non limiting embodiment of the invention, both layers 16, 17 and membrane 18 are at least partially positioned inside housing seat 21.

In a preferred but non limiting embodiment of the invention, first covering layer 16, second covering layer 17 and closing membrane 18 have a respective perimetral end position 35 arranged inside the housing seat 21 along all the closed outline.

Further, as it is visible in the attached figures, tank 1 comprises a predetermined quantity of filling material 22 positioned inside the housing seat 21 on the respective end portions of first covering layer 16, second covering layer 17, and closing membrane 18; filling material 22 positioned on said end portions 35 is configured for generating a thrust against said portions, which enables to lock layers 16, 17 and membrane 18.

Filling material comprises at least one of the following materials: ground, ballast, concrete, cement-stabilized sand.

### Biogas producing plant

Further, the invention refers to a biogas producing plant 100. Plant 100 comprises at least one first circuit 101 developing between an inlet 101a and outlet 101b; first circuit 101 is configured for enabling the organic matter to circulate from said inlet 101a to said outlet 101b.

As it is visible in the schematic view of Figure 1, inlet 101a of circuit 101 is positioned substantially at a loading area 103 receiving a predetermined quantity of organic matter used for producing biogas. Organic matter can comprise a substantially solid material, for example waste and shovelable into processing tanks or can comprise liquid substances, for example sewage.

As it is visible in Figure 1, the plant comprises at least one tank 1 fluidically communicating with first circuit 101 and configured for enabling to digest and/or store a predetermined quantity of organic matter; tank 1 is configured for generating biogas following an anaerobic bacterial fermentation of the organic matter.

In the preferred but non limiting embodiment of the invention, the plant 100 comprises: at least one tank 1 configured for defining a digester 105 adapted to enable to treat (ferment) the organic matter and at least one storage tank 1.

Storage tank is positioned between digestion tank 1 and outlet 101b of first circuit 101; storage tank 1 is configured for receiving the processed organic matter exiting the digesting tank 1, and ensuring a complete fermentation of the matter. Organic matter exiting storage tank 1 is delivered to a downloading area 104. The biomass exiting the storage tanks can be also reused for example as solid fertilizer.

Further, as it is visible in the attached figures, the plant comprises at least one second circuit 102 fluidically communicating with at least the digestion tank and configured for enabling the biogas generated by said tank 1 to circulate. Preferably, second circuit 102 is fluidically communicating both with the digestion tank and the storage tank because also this latter is adapted to generate biogas.

More particularly, second circuit 102 develops from a plurality of inlets 102a, fluidically communicating with tanks 1, to one or more outlets adapted to enable to deliver the generated biogas to one or more users.

The users adapted to collect the biogas generated by the tank can comprise biogas processing units which are configured for generating, by this latter, electric and/or thermal energy.

### Process for forming a tank for biogas producing plants

Further, the invention refers to a process for forming the above described tank 1. The process comprises the following steps.

The process comprises the provision of the reinforcing elements 7 each having a reticular structure 8, preferably a monolithic one of plastics material, at least partially flexible. Each reinforcing element 7 can be obtained by a co-extrusion process of a first series of precursors intersecated by a second series of precursors. Further, the co-extrusion step comprises a step of stretching the second precursors to form said mono-stretched reticular structure comprising a series of said first elements 8a (not stretched or substantially not stretched) intersecated with said series of second stretched elements 8b. Further, reticular structure 8 of reinforcing element 7 can be obtained by drilling a plastics material plate which is then stretched.

A further process step consists of providing the ground to be consolidated or the starting surface of the forming structure. This step comprises for example leveling a predetermined quantity of ground for forming base 3.

After the base 3 leveling step, the process comprises forming at least a first compacted ground layer 25 having a rising surface 33 and an upper abutment surface 34. During the process, the ground layer 25 is the first to be provided (layer 25 is at the lowermost level of the containing site 2) immediately after the base 3 leveling step; the process comprises, as a first layer providing step 25, providing the abutment surface 34 consisting in a further ground leveling to define a surface extending tangentially to the base 3: the abutment surface 34, in such case, is in the same development plane of base 3 as to substantially define an extension or prolongation of this latter.

The upper abutment surface 34 extends around base 3 to define a closed outline. After having formed the first ground layer 25, the process can provide the positioning of at least a plurality of formworks 28, for example of metal material, on the upper abutment surface of first ground layer 25. Formworks 28 are substantially positioned at the rising surface of ground layer 25 all around base 3 so that at least their envelope defines a closed outline around the base 3 itself. In a non limiting solution of the invention, formworks 28 are positioned in order to define at least partially the rising surface 33 of ground layers 25.

Then, the process provides a step of positioning, on the upper abutment surface 34 of first ground layer 25, a plurality of reinforcing elements 7: said plurality of reinforcing elements 7 extending around base 3 to define a closed outline. The step of positioning the reinforcing elements 7 can comprise a sub-step of partially overlapping the reinforcing elements 7 consecutively flanked on surface 34 so that these latter define, around base 3, a closed outline.

The step of positioning the reinforcing elements 7 is adapted to position first elements 8a of each reinforcing element 7 substantially along a development direction parallel or tangent to rising surface 33 at which said reinforcing element 7 is positioned.

Then, reinforcing elements 7 are connected to each other to define a continuous layer of elements adapted to cover at least partially the upper abutment surface 34 of first ground layer 25. Particularly, the step of positioning the reinforcing elements 7 comprises the sub-steps of spreading on the upper abutment surface 34 a first segment 13 of reinforcing element 7; such first segment 13 extends to the rising surface 33. Then, a front portion 27 of reinforcing element 7 is turned over to define at least one second segment 14 transversal to first segment 13. In order to keep the second segment 14 in a position transversal to first segment 13, said second segment 14 is engaged with the outer formwork 28. De facto, the front portion 27 is supported by the outer formwork 28.

After the above described steps, the process provides a step of positioning a second ground layer 26 so that the reinforcing elements 7 are at least partially interposed between the first and second ground layers 25, 26; second ground layer 26 having a respective rising surface 33 and a respective upper abutment surface 34: the upper abutment surface 34 extending around base 3 to define a closed outline.

De facto, the step of positioning the second ground layer 26 comprises a step of spreading a predetermined quantity of ground adapted to contact at least partially the first and second segments 13, 14 of reinforcing element 7. More particularly, such step provides spreading a portion of the predetermined ground quantity of first segment 13 of reinforcing element 7; then, such predetermined ground quantity is compacted. Such spreading and compacting steps are repeated until the compacted ground has the same height as second segment 14.

Then, the process provides a step of turning the front portion 27 of reinforcing element 7 over said second ground layer 26; the turning over step is suitable for arranging the reinforcing element 7 in a three-dimensional configuration suitable for defining, according to a transversal view of the side wall 4, a substantially "C" profile having a concavity facing away from the inner face 4a of side wall 4. More particularly, the step of turning over the front portion 27 is adapted to define the third segment 15 positioned on first segment 13; a predetermined quantity of compacted ground is interposed between said first and third segments 13, 15.

During the turning over step, first elements 8a are maintained parallel to each other and substantially horizontal and wherein said second elements 8b are positioned so that said outline has an open outline shape and/or at least one segment oriented transversally to the rising surface 33.

The above described steps are suitable for defining the reinforcing layer 9 formed by compacted ground and reinforcing elements 7; the process provides to repeat the above described steps to define a plurality of reinforced layers 9 overlapping each other so that these latter define the inner face 4a of side wall 4.

Further, the process can comprise a further step of forming the upper face 4b connected to the free edge 6 of side wall 4; the step of forming the upper face 4b comprises at least the following sub-steps: positioning a predetermined ground quantity on the last reinforced layer 9; leveling the predetermined ground quantity to define on the top of side wall 4 a flat abutment surface; compacting the predetermined leveled ground quantity. After the inner and upper face forming steps, the process can provide the formation of the outer face 4c.

After the above described steps, the process provides to form, at the upper face 4b, the trench 20. After the trench formation, the process provides to position and fix the first covering layer 16 at least on the inner face 4c of side wall 4; particularly, the step of positioning the first layer 16 is adapted to cover the whole inner face 4a of side wall: the perimetral end portion 35 of first layer is at least partially inserted inside the housing seat 21 of trench 20. Then, the process provides to position and fix the second layer 17 on first layer 16 to cover the whole base 3 and the whole inner face 4a. Also in this case, the perimetral end portion 35 of second layer 17 is inserted at least partially inside the housing seat 21 of trench 20 on the first layer 16.

Then, the process provides to position and fix the closing membrane 18 on the first and second layers 16 and 17. The membrane 18 is fixed to the side wall 4 so that the containing cavity 5 defines with the containing site 2 a closed volume. Also in this case, the perimetral end portion 35 of membrane 18 is inserted at least partially inside the housing seat 21 of trench 20 on the first and second layers 16, 17.

After the above described steps, the process provides a step of fixing the layers 16, 17 and membrane 18 to side wall. The fixing step provides to spread a predetermined quantity of filling material 22 inside the housing seat 21, particularly on the perimetral end portions therein contained.

### ADVANTAGES OF THE INVENTION

The present invention accomplishes substantial advantages. Particularly, the step of forming a digestion and/or storage tank 1 of ground enables to define an extremely flexible containing structure adaptable to possible sagging/settings of the ground. Such characteristic prevents the tank from having undesired breaches of the wall which could cause leakages or loss from the tank itself.

Further, the fact of forming a tank made of ground enables to substantially reduce the local environmental impact which is often high for tanks made of reinforced concrete and/or steel.

The particular ground structure enables to define on the upper surface of side wall 4 the above described trench; such method of fixing the layers 16, 17 but above all membrane 18, enables to lock such elements so that these latter are not cut or modified. Any change to the membrane fixing portions (for example the formation of notches) defines a weakening point of the membrane itself which tears/slits can develop from. De facto, the above described fixation (trench fixation) enables to provide a tank for biogas plants suitable for ensuring tightness against leakages or seepages; particularly, a tank suitable for ensuring an optimal tightness of a membrane covering the tank without causing undesired stresses which could damage the membrane itself. The particular fixation, besides avoiding to process the membrane, enables to define a locking with uniformly distributed stresses on all the perimetral end portions of membrane (also of the first and second layers 16, 17): such condition ensures an optimal and uniform tightness of the membrane (of layers) without causing high stresses which could damage the membrane itself. It is noted the tank is obtained by low cost materials - the ground is taken from a construction site while the plastics nets have an extremely reduced cost with respect to reinforced concrete and/or steel - in order to reduce the total cost of the plant.

The simplicity of forming the tank enables to reduce at a minimum the installation and disposal costs of the structure.

### LIST OF PARTS

- 1: tank
- 2: containing site
- 3: base
- 4: side wall
- 4a: inner face
- 4b: upper face
- 4c: outer face
- 5: containing cavity
- 6: free edge
- 7: reinforcing element
- 8: reticular structure
- 9: reinforcing layer
- 10: perimetral inner edge
- 11: perimetral outer edge
- 12: reference outer plane
- 13: first segment
- 14: second segment
- 15: third segment
- 16: first covering layer
- 17: second covering layer
- 18: closing membrane
- 19: net
- 20: trench
- 21: housing seat
- 22: filling material
- 23: bottom surface
- 24: edge
- 25: first ground layer
- 26: second ground layer
- 27: front portion
- 28: formwork
- 30: perimetral end portion of first layer 16
- 31: perimetral end portion of second layer 17
- 32: perimetral end portion of membrane
- 33: rising surface
- 34: upper abutment surface
- 35: perimetral end portion
- 100: plant
- 101: first circuit
- 101a: inlet
- 101b: outlet
- 102: second circuit
- 102a: inlet
- 102b: outlet
- 103: loading area
- 104: downloading area
- 105: digester

## Claims

1. Process for forming a tank (1) for biogas plants, comprising at least the following steps:
- providing a plurality of reinforcing elements (7), each of them having a reticular structure (8), each reinforcing element (7) comprising:
• a plurality of first elements (8a) spaced from each other and having an elongated shape according to a respective main extension direction;
• a plurality of second elements (8b) spaced from each other, which substantially extend along a direction transversal of said first elements (8a), second elements (8b) having an elongated shape according to a respective main extension direction;
- providing a predetermined quantity of ground;
- forming a containing site (2) comprising at least one base (3) and at least one side wall (4) mainly of ground, transversally emerging from base (3), side wall (4) having an inner face (4a) which, together with the base (3), defines a containing cavity (5), having a closed outline, arranged to receive a predetermined quantity of organic matter configured to generate a biogas following an anaerobic bacterial fermentation, the containing site (2) further comprising a free edge (6) placed opposite in respect to the base (3) and delimiting an opening of the containing cavity (5);
**characterized by** the fact the step of providing the ground side wall (4) comprises at least the following sub-steps:
• forming a first ground compacted layer (25) having a rising surface (33) and an upper abutment surface (34), the upper abutment surface (34) extending around the base (3) to define a closed outline;
• positioning on the upper abutment surface (34) of the first ground layer (25), a plurality of reinforcing elements (7), said plurality of reinforcing elements (7) extending around the base (3) to define a closed outline;
• connecting to each other said reinforcing elements (7) to define a continuous layer of elements suitable for covering at least partially the upper abutment surface (34) of the first ground layer (25);
• after having positioned the reinforcing elements (7), positioning a second ground layer (26), so that the reinforcing elements (7) are at least partially interposed between the first and second ground layers (25; 26), said second ground layer (26) having a corresponding rising surface (33) and a corresponding upper abutment surface (34), the upper abutment surface (34) extending around the base (3) to define a closed outline;
each ground layer and the respective reinforcing elements (7) associated to it, defining a reinforced layer (9), the process provides to repeat said sub-steps to define a plurality of reinforced layers (9) overlapping each other so that these latter define the inner face (4a) of side wall (4).

2. Process according to the preceding claim, wherein the reticular structure (8) has a monolithic structure at least partially flexible of plastics material.

3. Process according to claim 1 or 2, wherein the step of positioning each reinforcing element (7), comprises at least the following steps:
• spreading on the upper abutment surface (34) a first segment (13) of the reinforcing element (7);
• raising a front portion (27) of the reinforcing element (7) to define at least a second segment(14) transversal of first segment (13);
the step of positioning the second ground layer (26) comprises a step of spreading a predetermined quantity of ground suitable for contacting at least partially the first and second segments (13; 14) of the reinforcing element (7),
and wherein the step of spreading the filling material to cover at least partially said reinforcing element (7), comprises at least the following sub-steps:
• spreading a portion of the predetermined quantity of material on the first segment (13) of the reinforcing element (7);
• compacting the portion of the spread material;
• repeating the steps of spreading and compacting until it is reached the height of the second segment (14) of the reinforcing element (7).

4. Process according to anyone of the preceding claims, wherein the step of providing the side wall (4) comprises, after the step of positioning the second ground layer (26), a step of turning a front portion (27) of the reinforcing element (7) over said second ground layer (26), the turning over step being suitable to place the reinforcing element (7) in a tri-dimensional configuration suitable for defining, along a transversal view of the side wall (4), a substantially "C" profile having its concavity facing opposite with respect to the inner face (4a) of the side wall (4), and wherein the step of turning over the front portion (27) of the reinforcing element (7) is suitable for defining a third segment (15) placed on the second ground layer (26) which is interposed between the first and third segments (13; 15), the first and third segments (13; 15) being spaced from and substantially parallel to each other, second segment (14) linking said first and third segments (13; 15) and being transversal of these latter, particularly said second segment (14) extending substantially parallel to the inner face (4a) of side wall (4).

5. Process according to anyone of the preceding claims, wherein the step of positioning the reinforcing elements (7), comprises a sub-step of partially overlapping the reinforcing elements (7) of the same layer (9) so that these latter define, around the base (3), a closed outline.

6. Process according to anyone of the preceding claims, wherein the step of forming the containing site (2), is configured to define a free edge (6) substantially extending along a prevalent development plane, said containing site (2) having a height, defined by the minimum distance between the prevalent development plane of the free edge (6) and the base (3), comprised between 5 m and 15 m, particularly between 6 m and 12 m, still more particularly between 6 m and 10 m, the containing site (2) height substantially coinciding with the sum of the heights of the reinforced layers (9) forming the side wall (4).

7. Process according to anyone of the preceding claims, wherein the step of providing the side wall (4) comprises a further step of forming an upper face (4b) connected to the free edge (6) of side wall (4), transversally extending from the latter away respect the base (3) along all the perimeter of the side wall (4) itself, the step of providing the upper face (4b) comprising at least the following sub-steps:
• positioning a predetermined quantity of ground on the last reinforcing layer (9);
• leveling the predetermined quantity of ground for defining on the top of the side wall (4), a flat abutment surface;
• compacting the leveled predetermined quantity of ground.

8. Process according to anyone of the preceding claims, wherein the step of providing the reinforcing elements (7) comprises at least the following steps:
• co-extruding a series of first and second elements (8a; 8b) of plastics material to define a monolithic reticular structure of rectangular or square meshes;
• stretching the monolithic reticular structure along the development direction of second elements (8b) to obtain a mono-stretched reticular structure;
and wherein, after the step of co-extruding the first and second elements (8a; 8b), said first elements (8a) are not stretched or have a stretch ratio less than the one of the second elements (8b), the stretch ratio of an element being defined as the ratio of the final length of the element itself once it has been stretched to the starting length of such element before the action of stretching.

9. Process according to anyone of the preceding claims, comprising at least a further step of positioning a first covering layer (16) at least on the inner face (4a) of the side wall (4), said first covering layer (16) comprising at least one reticular structure net (19),
and wherein said process comprises at least a further step of positioning a second covering layer (17) at least on the inner face (4a) of the side wall (4) and on base (3), said step of providing the second covering layer (17) being after the step of positioning the first covering layer (16), said second covering layer (17) being positioned on first covering layer (16) so that the latter is interposed between the inner face (4a) and second covering layer (17),
and wherein said process comprises a further step of positioning a closing membrane (18) engaged to the containing site (2) and configured to hermetically close the containing cavity (5).

10. Process according to the preceding claim, comprising, after the step of forming the side wall (4) of reinforced ground, a step of forming at least one trench (20) at the inner face (4b) of side wall (4), said trench (20) having an housing seat (21) developing around the opening of the containing cavity (5) to define a closed outline, said housing seat (21) having an opening facing opposite with respect to the base (3);
and wherein said process further comprises a step of fixing the first covering layer (16), the second covering layer (17) and the closing membrane (18), said fixing step comprising at least the following steps:
- defining said trench (20) all around the opening of containing cavity (5) to define a closed outline housing seat (21);
- positioning a perimetral end portion (30) of first covering layer (16) inside the housing seat (21) of trench (20);
- positioning a perimetral end portion (31) of second covering layer (17) inside the housing seat (21) of trench (20) on first covering layer (16);
- positioning a perimetral end portion (32) of closing membrane (18) inside the housing seat (21) of trench (20) on second covering layer (17);
- filling the housing seat (21) of trench (20) with a predetermined quantity of filling material after the preceding steps of positioning the covering layers (16; 17) and membrane (18).

11. Tank (1) for biogas producing plants, comprising:
- at least a containing site (2) at least partially made of reinforced ground, comprising at least a base (3) and a side wall (4) of ground transversally emerging from base (3), said side wall (3) having at least one inner face (4a) which, together with the base (3), defines a containing cavity (5) arranged to receive a predetermined quantity of organic matter configured to produce a biogas following an anaerobic bacterial fermentation, containing site (2) comprising a free edge (6) placed opposite with respect to the base (3) and defining an opening of the containing cavity (5);
- a plurality of reinforcing elements (7), each of them having a reticular structure (8), each reinforcing element (7) comprising:
• a plurality of first elements (8a) spaced from each other, and having an elongated shape along a respective prevalent development direction,
• a plurality of second elements (8b) spaced from each other, which substantially develops in a direction transversal of said first elements (8a), second elements (b) having also an elongated shape along a respective prevalent development direction;
**characterized in that** said reinforcing elements (7) are placed outside the containing cavity (5), inside the side wall (4) at the inner face (4a),
at least some of said reinforcing elements (7) being connected to each other around the perimeter of the side wall (4) to define a continuous layer of reinforcing elements defining around the containing cavity (5) a closed outline, said continuous layer of reinforcing elements (7) together with the ground of side wall (4) defining a reinforcing layer (9), said reinforcing elements (7) being configured to define a plurality of reinforced layers (9) placed on each other to define said inner face (4a).

12. Tank according to the preceding claim, wherein the reticular structure (8) comprises a monolithic structure, at least partially flexible, of plastics material.

13. Tank according to anyone of the preceding claims of tank, wherein the side wall (4) comprises at least an upper face (4b) connected to the free edge (6) of side wall (4) extending transversally of the latter away with respect to the base (3) along all the perimeter of the side wall (4) itself;
and wherein side wall (4) comprises an outer face (4c) connected to upper face (4b) and transversally emerging with respect to the latter, said outer face (4c) emerging approaching the base (3) and away with respect to the inner face (4a), upper face (4b) being interposed between inner face (4a) and outer face (4c).

14. Tank according to the preceding claim, wherein second elements (8b) of reinforcing element (7), after having being formed, are stretched along their prevalent development direction, said first elements (8a) are not stretched or have a stretch ratio less than, optionally at least half, the one of second elements (8b), stretch ratio of an element being defined as the ratio of the final length of the element itself once it has been stretched to the starting length of the element itself before having being stretched;
and wherein second elements (8b) have a stretch ratio greater than 3, optionally comprised between 4 and 10, more optionally between 5 and 8, stretch ratio of second elements being defined as the ratio of a final length of second elements after having stretched the same to the starting length of second elements before stretching.

15. Tank according to anyone of the preceding claims of tank, wherein each reinforcing element (7) is placed according to a tri-dimensional configuration suitable for defining, along a transversal view of side wall (4), a substantially "C" profile having a concavity facing on the opposite side the inner face (4a) of side wall (4);
and wherein the open outline reinforcing element (7) has at least a first segment (13), second segment (14) and third segment (15), said first and third segments (13; 15) being spaced from and substantially parallel to each other, said second segment (14) linking said first and third segments (13; 15) and being transversal of these latter, particularly said second lengths (14) extending substantially parallel to inner face (4a) of side wall (4).

16. Plant for producing a biogas (100), comprising:
- at least one first circuit (101) developing between an inlet (101a) and outlet (101b), said first circuit (101) being configured to enable an organic matter to circulate from said inlet (101a) to said outlet (101b);
- at least a tank (1) according to anyone of the preceding claims of tank, said tank (1) being in fluid communication with first circuit (101) and configured to enable to digest and/or store a predetermined quantity of organic matter, said tank (1) being configured to generate a biogas following an anaerobic bacterial fermentation of the organic matter;
- at least a second circuit (102) in fluid communication with said tank (1) and configured to enable the circulation of biogas generated from said tank.

## Patentansprüche

1. Verfahren zum Bilden eines Behälters (1) für Biogasanlagen, umfassend wenigstens die folgenden Schritte:
• Bereitstellen einer Mehrzahl von Verstärkungselementen (7), von denen jedes eine retikuläre Struktur (8) aufweist, wobei jedes Verstärkungselement (7) umfasst:
• eine Mehrzahl erster Elemente (8a), welche voneinander beabstandet sind und eine längliche Form gemäß einer jeweiligen Haupterstreckungsrichtung aufweisen;
• eine Mehrzahl zweiter voneinander beabstandeter Elemente (8b), welche sich im Wesentlichen entlang einer zu den ersten Elementen (8a) transversalen Richtung erstrecken, wobei die zweiten Elemente (8b) eine längliche Form gemäß einer jeweiligen Haupterstreckungsrichtung aufweisen;
• Bereitstellen einer vorbestimmten Menge von Boden;
• Bilden einer Aufnahmestelle (2), welche wenigstens eine Basis (3) und wenigstens eine Seitenwand (4) hauptsächlich aus Boden umfasst, welche transversal aus der Basis (3) hervorgeht, wobei die Seitenwand (4) eine innere Fläche (4a) aufweist, welche zusammen mit der Basis (3) einen Aufnahmehohlraum (5) definiert, welcher einen geschlossenen Umriss aufweist und angeordnet ist, um eine vorbestimmte Menge einer organischen Substanz aufzunehmen, welche dazu eingerichtet ist, einer anaeroben bakteriellen Fermentation folgend ein Biogas zu erzeugen, wobei die Aufnahmestelle (2) ferner einen freien Rand (6) umfasst, welcher in Bezug auf die Basis (3) entgegengesetzt platziert ist und eine Öffnung des Aufnahmehohlraums (5) begrenzt;
**gekennzeichnet durch** die Tatsache, dass der Schritt des Bereitstellens der Bodenseitenwand (4) wenigstens die folgenden Unterschritte umfasst:
• Bilden einer ersten verdichteten Bodenschicht (25), welche eine ansteigende Fläche (33) und eine obere Anlagefläche (34) aufweist, wobei sich die obere Anlagefläche (34) um die Basis (3) erstreckt, um einen geschlossenen Umriss zu definieren;
• Positionieren, an der oberen Anlagefläche (34) der ersten Bodenschicht (25), einer Mehrzahl von Verstärkungselementen (7), wobei sich die Mehrzahl von Verstärkungselementen (7) um die Basis (3) erstreckt, um einen geschlossenen Umriss zu definieren;
• Verbinden der Verstärkungselemente (7) miteinander, um eine kontinuierliche Schicht von Elementen zu definieren, welche dazu geeignet sind, die obere Anlagefläche (34) der ersten Bodenschicht (25) wenigstens teilweise zu bedecken;
• nachdem die Verstärkungselemente (7) positioniert worden sind, Positionieren einer zweiten Bodenschicht (26), so dass die Verstärkungselemente (7) wenigstens teilweise zwischen der ersten und der zweiten Bodenschicht (25; 26) eingefügt sind, wobei die zweite Bodenschicht (26) eine entsprechende ansteigende Fläche (33) und eine entsprechende obere Anlagefläche (34) aufweist, wobei sich die obere Anlagefläche (34) um die Basis (3) erstreckt, um einen geschlossenen Umriss zu definieren;
wobei jede Bodenschicht und die jeweiligen damit assoziierten Verstärkungselemente (7) eine Verstärkungsschicht (9) definieren, wobei das Verfahren vorsieht, die Unterschritte zu wiederholen, um eine Mehrzahl von Verstärkungsschichten (9) zu definieren, welche einander überlappen, so dass die Letzteren die Innenfläche (4a) der Seitenwand (4) definieren.

2. Verfahren nach dem vorhergehenden Anspruch, wobei die retikuläre Struktur (8) eine wenigstens teilweise flexible monolithische Struktur aus Kunststoffmaterial aufweist.

3. Verfahren nach Anspruch 1 oder 2, wobei der Schritt des Positionierens jedes Verstärkungselements (7) wenigstens die folgenden Schritte umfasst:
• Ausbreiten an der oberen Anlagefläche (34) eines ersten Segments (13) des Verstärkungselements (7);
• Anheben eines vorderen Abschnitts (27) des Verstärkungselements (7), um wenigstens ein zweites Segment transversal des ersten Segments (13) zu definieren;
wobei der Schritt des Positionierens der zweiten Bodenschicht (26) einen Schritt eines Ausbreitens einer vorbestimmten Menge von Boden umfasst, welcher dazu geeignet ist, das erste und das zweite Segment (13; 14) des Verstärkungselements (7) wenigstens teilweise zu berühren,
und wobei der Schritt des Ausbreitens des Füllmaterials, um das Verstärkungselement (7) wenigstens teilweise abzudecken, wenigstens die folgenden Unterschritte umfasst:
• Ausbreiten eines Abschnitts der vorbestimmten Menge eines Materials an dem ersten Segment (13) des Verstärkungselements (7);
• Verdichten des Abschnitts des ausgebreiteten Materials;
• Wiederholen der Schritte des Ausbreitens und Verdichtens bis die Höhe des zweiten Segments (14) des Verstärkungselements (7) erreicht ist.

4. Verfahren nach einem der vorhergehenden Ansprüche, wobei der Schritt des Bereitstellens der Seitenwand (4), nach dem Schritt des Positionierens der zweiten Bodenschicht (26), einen Schritt eines Wendens eines vorderen Abschnitts (27) des Verstärkungselements (7) über die zweite Bodenschicht (26) umfasst, wobei der Schritt des Wendens dazu geeignet ist, das Verstärkungselement (7) in einer dreidimensionalen Konfiguration zu platzieren, welche dazu geeignet ist, entlang einer transversalen Ansicht der Seitenwand (4), ein im Wesentlichen "C"-förmiges Profil zu definieren, welches seine Konkavität entgegengesetzt in Bezug auf die Innenfläche (4a) der Seitenwand (4) aufweist, und wobei der Schritt des Wendens des vorderen Abschnitts (27) des Verstärkungselements (7) dazu geeignet ist, ein drittes an der zweiten Bodenschicht (26) positioniertes Segment (15) zu definieren, welches zwischen dem ersten und dem dritten Segment (13; 15) eingefügt ist, wobei das erste und das dritte Segment (13; 15) voneinander beabstandet und im Wesentlichen parallel zueinander sind, wobei das zweite Segment (14) das erste und das dritte Segment (13; 15) verbindet und transversal zu den letzteren ist, insbesondere wobei sich das zweite Segment (14) im Wesentlichen parallel zu der Innenfläche (4a) der Seitenwand (4) erstreckt.

5. Verfahren nach einem der vorhergehenden Ansprüche, wobei der Schritt des Positionierens des Verstärkungselements (7) einen Unterschritt eines teilweisen Überlappens des Verstärkungselements (7) der gleichen Schicht (9) umfasst, so dass die Letzteren um die Basis (3) einen geschlossenen Umriss definieren.

6. Verfahren nach einem der vorhergehenden Ansprüche, wobei der Schritt des Bildens der Aufnahmestelle (2) dazu eingerichtet ist, einen freien Rand (6) zu definieren, welcher sich im Wesentlichen entlang einer vorherrschenden Entwicklungsebene erstreckt, wobei die Aufnahmestelle (2) eine Höhe aufweist, welche durch den minimalen Abstand zwischen der vorherrschenden Entwicklungsebene des freien Randes (6) und der Basis (3) definiert ist, zwischen 5 m und 15 m, insbesondere zwischen 6 m und 12m, noch spezieller zwischen 6 m und 10 umfasst ist, wobei die Höhe der Aufnahmestelle (2) im Wesentlichen mit der Summe der Höhen der Verstärkungsschichten (9) koinzidiert, welche die Seitenwand (4) bilden.

7. Verfahren nach einem der vorhergehenden Ansprüche, wobei der Schritt des Bereitstellens der Seitenwand (4) einen weiteren Schritt eines Bildens einer oberen Fläche (4b) umfasst, welche mit dem freien Rand (6) der Seitenwand (4) verbunden ist, sich von dem Letzteren weg in Bezug auf die Basis (3) entlang des gesamten Umfangs der Seitenwand (4) selbst transversal erstreckend, wobei der Schritt des Bereitstellens der oberen Fläche (4b) wenigstens die folgenden Unterschritte umfasst:
• Positionieren einer vorbestimmten Menge von Boden an der letzten Verstärkungsschicht (9);
• Nivellieren der vorbestimmten Menge von Boden zum Definieren einer ebenen Anlagefläche an der Oberseite der Seitenwand (4);
• Verdichten der nivellierten vorbestimmten Menge von Boden.

8. Verfahren nach einem der vorhergehenden Ansprüche, wobei der Schritt des Bereitstellens der Verstärkungselemente (7) wenigstens die folgenden Schritte umfasst:
• Co-Extrudieren einer Reihe erster und zweiter Elemente (8a; 8b) aus Kunststoffmaterial, um eine monolithische retikuläre Struktur rechtwinkliger oder quadratischer Maschen zu definieren;
• Dehnen der monolithischen retikulären Struktur entlang der Entwicklungsrichtung zweiter Elemente (8b), um eine mono-gedehnte rechtwinklige Struktur zu erhalten;
und wobei, nach dem Schritt des Co-Extrudierens der ersten und der zweiten Elemente (8a; 8b), die ersten Elemente (8a) nicht gedehnt sind oder ein Dehnungsverhältnis aufweisen, welches niedriger als das der zweiten Elemente (8b) ist, wobei das Dehnungsverhältnis eines Elements als das Verhältnis der Endlänge des Elements selbst, sobald es gedehnt worden ist, zu der Startlänge eines solchen Elements vor dem Dehnvorgang definiert ist.

9. Verfahren nach einem der vorhergehenden Ansprüche, umfassend wenigstens einen weiteren Schritt eines Positionierens einer ersten Deckschicht (16) wenigstens an der Innenfläche (4a) der Seitenwand (4), wobei die erste Deckschicht (16) wenigstens ein rechtwinkliges Strukturnetz (19) umfasst,
und wobei das Verfahren wenigstens einen weiteren Schritt eines Positionierens einer zweiten Deckschicht (17) wenigstens an der Innenfläche (4a) der Seitenwand (4) und an der Basis (3) umfasst, wobei der Schritt des Bereitstellens der zweiten Deckschicht (17) nach dem Schritt des Positionierens der ersten Deckschicht (16) erfolgt, wobei die zweite Deckschicht (17) an der ersten Deckschicht (16) positioniert wird, so dass letztere zwischen der Innenfläche (4a) und der zweiten Deckschicht (17) eingefügt ist,
und wobei das Verfahren einen weiteren Schritt einen Positionierens einer Verschlussmembran (18) umfasst, welche mit der Aufnahmestelle (2) in Eingriff steht und dazu eingerichtet ist, den Aufnahmehohlraum (5) hermetisch zu verschließen.

10. Verfahren nach dem vorhergehenden Anspruch, umfassend, nach dem Schritt des Bildens der Seitenwand (4) aus verstärktem Boden, einen Schritt eines Bildens wenigstens eines Grabens (20) an der Innenfläche (4b) der Seitenwand (4), wobei der Graben (20) einen Gehäusesitz (21) aufweist, welcher sich um die Öffnung des Aufnahmehohlraums (5) erstreckt, um einen geschlossenen Umriss zu definieren, wobei der Gehäusesitz (21) eine Öffnung aufweist, welche in Bezug auf die Basis (3) entgegengesetzt ist;
und wobei das Verfahren ferner einen Schritt eines Fixierens der ersten Deckschicht (16), der zweiten Deckschicht (17) und der Verschlussmembran (18) umfasst, wobei der Fixierschritt wenigstens die folgenden Schritte umfasst:
- Definieren des Grabens (20) um die Öffnung des Aufnahmehohlraums (5) herum, um einen Gehäusesitz (21) mit geschlossenem Umriss zu definieren;
- Positionieren eines perimetralen Endabschnitts (30) der ersten Deckschicht (16) innerhalb des Gehäusesitzes (21) des Grabens (20);
- Positionieren eines perimetralen Endabschnitts (31) der zweiten Deckschicht (17) innerhalb des Gehäusesitzes (21) des Grabens (20) an der ersten Deckschicht (16);
- Positionieren eines perimetralen Endabschnitts (32) der Verschlussmembran (18) innerhalb des Gehäusesitzes (21) des Grabens (20) an der zweiten Deckschicht (17);
- Füllen des Gehäusesitzes (21) des Grabens (20) mit einer vorbestimmten Menge eines Füllmaterials nach den vorhergehenden Schritten des Positionierens der Deckschichten (16; 17) und der Membran (18).

11. Behälter (1)für Biogaserzeugungsanlagen, umfassend:
- wenigstens eine Aufnahmestelle (2) welche wenigstens teilweise aus verstärktem Boden hergestellt ist, welche wenigstens eine Basis (3) und eine Seitenwand (4) aus Boden umfasst, welche transversal aus der Basis (3) hervorgeht, wobei die Seitenwand (3) wenigstens eine innere Fläche (4a) umfasst, welche zusammen mit der Basis (3) einen Aufnahmehohlraum (5) definiert, welcher angeordnet ist, um eine vorbestimmte Menge einer organischen Substanz aufzunehmen, welche dazu eingerichtet ist, einer anaeroben bakteriellen Fermentation folgend ein Biogas zu erzeugen, wobei die Aufnahmestelle (2) einen freien Rand (6) umfasst, welcher in Bezug auf die Basis (3) entgegengesetzt platziert ist und eine Öffnung des Aufnahmehohlraums (5) definiert;
- eine Mehrzahl von Verstärkungselementen (7), von denen jedes eine retikuläre Struktur (8) aufweist, wobei jedes Verstärkungselement (7) umfasst:
• eine Mehrzahl erster Elemente (8a), welche voneinander beabstandet sind und eine längliche Form entlang einer jeweiligen vorherrschenden Entwicklungsrichtung aufweisen;
• eine Mehrzahl zweiter voneinander beabstandeter Elemente (8b), welche sich im Wesentlichen in einer zu den ersten Elementen (8a) transversalen Richtung entwickelt, wobei die zweiten Elemente (b) ebenfalls eine längliche Form entlang einer jeweiligen vorherrschenden Entwicklungsrichtung aufweisen;
**dadurch gekennzeichnet, dass** die Verstärkungselemente (7) außerhalb des Aufnahmehohlraums (5) innerhalb der Seitenwand (4) an der Innenfläche (4a) platziert sind,
wobei wenigstens einige der Verstärkungselemente (7) um den Umfang der Seitenwand (4) miteinander verbunden sind, um eine kontinuierliche Schicht von Verstärkungselementen zu definieren, welche um den Aufnahmehohlraum (5) einen geschlossenen Umriss definieren, wobei die kontinuierliche Schicht von Verstärkungselementen (7) zusammen mit dem Boden der Seitenwand (4) eine Verstärkungsschicht (9) definiert, wobei die Verstärkungselemente (7) dazu eingerichtet sind, eine Mehrzahl von Verstärkungsschichten (9) zu definieren, welche aufeinander platziert sind, um die Innenfläche (4a) zu definieren.

12. Behälter nach dem vorhergehenden Anspruch, wobei die retikuläre Struktur (8) eine wenigstens teilweise flexible monolithische Struktur aus Kunststoffmaterial umfasst.

13. Behälter nach einem der vorgehenden Ansprüche des Behälters, wobei die Seitenwand (4) wenigstens eine obere Fläche (4b) umfasst, welche mit dem freien Rand (6) der Seitenwand (4) verbunden ist, sich von der Letzteren weg in Bezug auf die Basis (3) entlang des gesamten Umfangs der Seitenwand (4) selbst transversal erstreckend,
und wobei die Seitenwand (4) eine äußere Fläche (4c) umfasst, welche mit der oberen Fläche (4b) verbunden ist und aus der Letzteren transversal hervorgeht, wobei die äußere Fläche (4c) sich der Basis (3) annähernd und weg in Bezug auf die innere Fläche (4a) hervorgeht, wobei die obere Fläche (4b) zwischen der inneren Fläche (4a) und der äußeren Fläche (4c) eingefügt ist.

14. Behälter nach dem vorhergehenden Anspruch, wobei die zweiten Elemente (8b) des Verstärkungselements (7), nachdem sie gebildet worden sind, entlang ihrer vorherrschenden Entwicklungsrichtung gedehnt sind, wobei die ersten Elemente (8a) nicht gedehnt sind oder ein Dehnungsverhältnis aufweisen, welches niedriger als, optional wenigstens die Hälfte, dasjenige/desjenigen der zweiten Elemente (8b) ist, wobei das Dehnungsverhältnis eines Elements als das Verhältnis der Endlänge des Elements selbst, sobald es gedehnt worden ist, zu der Startlänge des Elements selbst, bevor es gedehnt worden ist, definiert ist;
und wobei die zweiten Elemente (8b) ein Dehnungsverhältnis von mehr als 3, optional umfasst zwischen 4 und 10, weiter optional zwischen 5 und 8 aufweisen, wobei ein Dehnungsverhältnis der zweiten Elemente als das Verhältnis einer Endlänge der zweiten Elemente, nachdem dieselben gedehnt worden sind, zu der Startlänge der zweiten Elemente, vor einem Dehnen, definiert ist.

15. Behälter nach einem der vorhergehenden Ansprüche des Behälters, wobei jedes Verstärkungselement (7) gemäß einer dreidimensionalen Konfiguration platziert ist, welche dazu geeignet ist, entlang einer transversalen Ansicht der Seitenwand (4), ein im Wesentlichen "C"-förmiges Profil zu definieren, welches eine Konkavität aufweist, welche an der entgegengesetzten Seite der Innenfläche (4a) der Seitenwand (4) zugewandt ist, und wobei das Verstärkungselement (7) mit einem offenen Umriss, wenigstens ein erstes Segment (13), ein zweites Segment (14) und ein drittes Segment (15) aufweist, wobei das erste und das dritte Segment (13; 15) voneinander beabstandet und im Wesentlichen parallel zueinander sind, wobei das zweite Segment (14) das erste und das dritte Segment (13; 15) verbindet und transversal zu den letzteren ist, insbesondere wobei sich das zweite Segment (14) im Wesentlichen parallel zu der Innenfläche (4a) der Seitenwand (4) erstreckt.

16. Anlage zum Herstellen eines Biogases (100), umfassend:
- wenigstens einen ersten Kreislauf (101), welcher sich zwischen einem Einlass (101a) und einem Auslass (101b) entwickelt, wobei der erste Kreislauf (101) dazu eingerichtet ist, einer organischen Substanz zu ermöglichen, von dem Einlass (101a) zu dem Auslass (101b) zu zirkulieren;
- wenigstens einen Behälter (1) nach einem der vorhergehenden Ansprüche des Behälters, wobei der Behälter in Fluidkommunikation mit dem ersten Kreislauf (101) steht und dazu eingerichtet ist, eine vorbestimmte Menge einer organischen Substanz zu digerieren und/oder zu speichern, wobei der Behälter (1) dazu eingerichtet ist, einer anaeroben bakteriellen Fermentation der organischen Substanz folgend ein Biogas zu erzeugen;
- wenigstens einen zweiten Kreislauf (102), welcher in Fluidkommunikation mit dem Behälter (1) steht und dazu eingerichtet ist, die Zirkulation von aus dem Behälter erzeugtem Biogas zu ermöglichen.

## Revendications

1. Procédé de formation d'une cuve (1) pour installations de biogaz, comprenant au moins les étapes suivantes :
- de fourniture d'une pluralité d'éléments de renfort (7), dont chacun présente une structure réticulaire (8), chaque élément de renfort (7) comprenant :
• une pluralité de premiers éléments (8a) espacés l'un de l'autre et ayant une forme allongée selon un sens d'extension principal respectif ;
• une pluralité de seconds éléments (8b) espacés l'un de l'autre, qui s'étendent sensiblement le long d'un sens transversal auxdits premiers éléments (8a), de seconds éléments (8b) ayant une forme allongée selon un sens d'extension principal respectif ;
- de fourniture d'une quantité prédéterminée de sol ;
- de formation d'un site de retenue (2) comprenant au moins une base (3) et au moins une paroi latérale (4) principalement de sol, émergeant transversalement depuis la base (3), la paroi latérale (4) ayant une face interne (4a) qui, conjointement à la base (3), définit une cavité de retenue (5), ayant un contour fermé, configurée pour recevoir une quantité prédéterminée de matière organique configurée pour générer un biogaz suite à une fermentation bactérienne anaérobie, le site de retenue (2) comprenant en outre un bord libre (6) placé opposé par rapport à la base (3) et délimitant une ouverture de la cavité de retenue (5) ;
**caractérisé par le fait que** l'étape de fourniture de la paroi latérale de sol (4) comprend au moins les sous-étapes suivantes :
• formation d'une première couche compactée de sol (25) ayant une surface d'élévation (33) et une surface de butée supérieure (34), la surface de butée supérieure (34) s'étendant autour de la base (3) pour définir un contour fermé ;
• positionnement sur la surface de butée supérieure (34) de la première couche de sol (25), d'une pluralité d'éléments de renfort (7), ladite pluralité d'éléments de renfort (7) s'étendant autour de la base (3) pour définir un contour fermé ;
• de connexion les uns aux autres desdits éléments de renfort (7) pour définir une couche continue d'éléments convenant pour recouvrir au moins partiellement la surface de butée supérieure (34) de la première couche de sol (25) ;
• après avoir positionné les éléments de renfort (7), le positionnement d'une seconde couche de sol (26), de sorte que les éléments de renfort (7) sont au moins partiellement interposés entre la première et la seconde couche de sol (25 ; 26), ladite seconde couche de sol (26) ayant une surface d'élévation (33) correspondante et une surface de butée supérieure correspondante (34), la surface de butée supérieure (34) s'étendant autour de la base (3) pour définir un contour fermé ;
chaque couche de sol et les éléments de renfort respectifs (7) associés à celle-ci, définissant une couche renforcée (9), le procédé assurant la répétition desdites sous-étapes pour définir une pluralité de couches renforcées (9) se chevauchant l'une l'autre de sorte que ces dernières définissent la face interne (4a) de la paroi latérale (4).

2. Procédé selon la revendication précédente, la structure réticulaire (8) ayant une structure monolithique au moins partiellement souple en matière plastique.

3. Procédé selon la revendication 1 ou 2, l'étape de positionnement de chaque élément de renfort (7), comprenant au moins les étapes suivantes :
• étalement sur la surface de butée supérieure (34) d'un premier segment (13) de l'élément de renfort (7) ;
• élévation d'une partie avant (27) de l'élément de renfort (7) pour définir au moins un second segment (14) transversal au premier segment (13) ;
l'étape de positionnement de la seconde couche de sol (26) comprenant une étape d'étalement d'une quantité prédéterminée de sol convenant à la mise en contact au moins partiellement du premier et du second segment (13; 14) de l'élément de renfort (7),
et l'étape d'étalement du matériau de remplissage pour recouvrir au moins partiellement ledit élément de renfort (7), comprend au moins les sous-étapes suivantes :
• étalement d'une partie de la quantité prédéterminée du matériau sur le premier segment (13) de l'élément de renfort (7) ;
• compactage de la partie du matériau étalé ;
• répétition des étapes d'étalement et de compactage jusqu'à ce qu'il atteigne la hauteur du second segment (14) de l'élément de renfort (7).

4. Procédé selon l'une quelconque des revendications précédentes, l'étape de fourniture de la paroi latérale (4) comprend, après l'étape de positionnement de la seconde couche de sol (26), une étape consistant à tourner une partie avant (27) de l'élément de renfort (7) sur ladite seconde couche de sol (26), la sur-étape de tournage convenant pour placer l'élément de renfort (7) dans une configuration tridimensionnelle convenant pour définir, le long d'une vue transversale de la paroi latérale (4), un profil sensiblement en « C » ayant sa concavité faisant face à l'opposé par rapport à la face interne (4a) de la paroi latérale (4), et l'étape consistant à tourner par-dessus la partie avant (27) de l'élément de renfort (7) convenant pour définir un troisième segment (15) placé sur la seconde couche de sol (26) qui est interposée entre le premier et le troisième segment (13 ; 15), le premier et le troisième segment (13 ; 15) étant espacés, et sensiblement parallèles, l'un de l'autre, le second segment (14) liant lesdits premier et troisième segment (13 ; 15) et étant transversal à ces derniers, particulièrement ledit second segment (14) s'étendant sensiblement parallèle à la face interne (4a) de la paroi latérale (4).

5. Procédé selon l'une quelconque des revendications précédentes, l'étape de positionnement des éléments de renfort (7), comprenant une sous-étape de chevauchement partiellement des éléments de renfort (7) de la même couche (9) de sorte que ces dernières définissent, autour de la base (3), un contour fermé.

6. Procédé selon l'une quelconque des revendications précédentes, l'étape de formation du site de retenue (2), étant configurée pour définir un bord libre (6) s'étendant sensiblement le long d'un plan de développement prévalent, ledit site de retenue (2) ayant une hauteur, définie par la distance minimale entre le plan de développement prévalent du bord libre (6) et la base (3), comprise entre 5 m et 15 m, particulièrement entre 6 m et 12 m, encore plus particulièrement entre 6 m et 10 m, la hauteur du site de retenue (2) coïncidant sensiblement avec la somme des hauteurs des couches renforcées (9) formant la paroi latérale (4).

7. Procédé selon l'une quelconque des revendications précédentes, l'étape de fourniture de la paroi latérale (4) comprenant une étape supplémentaire de formation d'une face supérieure (4b) reliée au bord libre (6) de la paroi latérale (4), s'étendant transversalement depuis cette dernière par rapport à la base (3) le long de tout le périmètre de la paroi latérale (4) elle-même, l'étape de fourniture de la face supérieure (4b) comprenant au moins les sous-étapes suivantes :
• positionnement d'une quantité prédéterminée de sol sur la dernière couche de renfort (9) ;
• mise à niveau de la quantité prédéterminée de sol pour définir sur le dessus de la paroi latérale (4), une surface de butée plate ;
• compactage de la quantité prédéterminée mise à niveau du sol.

8. Procédé selon l'une quelconque des revendications précédentes, l'étape de fourniture des éléments de renfort (7) comprenant au moins les étapes suivantes :
• co-extrusion d'une série de premiers et seconds éléments (8a ; 8b) de matière plastique pour définir une structure réticulaire monolithique de mailles rectangulaires ou carrées ;
• étirage de la structure réticulaire monolithique le long du sens de développement des seconds éléments (8b) pour obtenir une structure réticulaire mono-étirée ;
et où, après l'étape de co-extrusion des premiers et seconds éléments (8a ; 8b), lesdits premiers éléments (8a) ne sont pas étirés ou présentent un rapport d'étirage inférieur à l'un des seconds éléments (8b), le rapport d'étirage d'un élément étant défini comme le rapport de la longueur finale de l'élément lui-même une fois qu'il a été étiré jusqu'à la longueur de départ d'un tel élément avant l'action d'étirage.

9. Procédé selon l'une quelconque des revendications précédentes, comprenant au moins une étape supplémentaire de positionnement d'une première couche de recouvrement (16) au moins sur la face interne (4a) de la paroi latérale (4), ladite première couche de recouvrement (16) comprenant au moins un filet à structure réticulaire (19),
et où ledit procédé comprend au moins une étape supplémentaire de positionnement d'une seconde couche de recouvrement (17) au moins sur la face interne (4a) de la paroi latérale (4) et sur la base (3), ladite étape de fourniture de la seconde couche de recouvrement (17) étant située après l'étape de positionnement de la première couche de recouvrement (16), ladite seconde couche de recouvrement (17) étant positionnée sur la première couche de recouvrement (16) de sorte que cette dernière est interposée entre la face interne (4a) et la seconde couche de recouvrement (17),
et où ledit procédé comprend une étape supplémentaire de positionnement d'une membrane de fermeture (18) engagée au niveau du site de retenue (2) et configurée pour fermer hermétiquement la cavité de retenue (5).

10. Procédé selon la revendication précédente, comprenant, après l'étape de formation de la paroi latérale (4) du sol renforcé, une étape de formation d'au moins l'une d'une tranchée (20) au niveau de la face interne (4b) de la paroi latérale (4), ladite tranchée (20) ayant une assise de logement (21) se développant autour de l'ouverture de la cavité de retenue (5) pour définir un contour fermé, ladite assise de logement (21) ayant une ouverture faisant face vers l'opposé de la base (3) ;
et ledit procédé comprenant en outre une étape de fixation de la première couche de recouvrement (16), de la seconde couche de recouvrement (17) et de la membrane de fermeture (18), ladite étape de fixation comprenant au moins les étapes suivantes :
- définition de ladite tranchée (20) tout autour de l'ouverture de la cavité de retenue (5) pour définir une assise de logement à contour fermé (21) ;
- positionnement d'une partie d'extrémité dans le périmètre (30) de la première couche de recouvrement (16) à l'intérieur de l'assise de logement (21) de la tranchée (20) ;
- positionnement d'une partie d'extrémité dans le périmètre (31) d'une seconde couche de recouvrement (17) à l'intérieur de l'assise de logement (21) de la tranchée (20) sur la première couche de recouvrement (16) ;
- positionnement d'une partie d'extrémité dans le périmètre (32) de la membrane de fermeture (18) à l'intérieur de l'assise de logement (21) de la tranchée (20) sur la seconde couche de recouvrement (17) ;
- remplissage de l'assise de logement (21) de la tranchée (20) avec une quantité prédéterminée de matériau de remplissage après les étapes précédentes de positionnement des couches de recouvrement (16 ; 17) et de la membrane (18).

11. Cuve (1) pour installations de production de biogaz, comprenant :
- au moins un site de retenue (2) au moins partiellement constitué de sol renforcé, comprenant au moins une base (3) et une paroi latérale (4) de sol émergeant transversalement à la base (3), ladite paroi latérale (3) ayant au moins une face interne (4a) qui, conjointement à la base (3), définit une cavité de retenue (5) disposée pour recevoir une quantité prédéterminée de matière organique configurée pour produire un biogaz suite à une fermentation bactérienne anaérobie, le site de retenue (2) comprenant un bord libre (6) placé à l'opposé par rapport à la base (3) et définissant une ouverture de la cavité de retenue (5) ;
une pluralité d'éléments de renfort (7), chacun ayant une structure réticulaire (8), chaque élément de renfort (7) comprenant :
• une pluralité de premiers éléments (8a) espacés l'un de l'autre, et ayant une forme allongée le long d'un sens de développement prévalent respectif,
• une pluralité de seconds éléments (8b) espacés l'un de l'autre, qui se développent sensiblement dans un sens transversal auxdits premiers éléments (8a), les seconds éléments (b) ayant également une forme allongée le long d'un sens de développement prévalent respectif ;
**caractérisée en ce que** lesdits éléments de renfort (7) sont placés à l'extérieur de la cavité de retenue (5), à l'intérieur de la paroi latérale (4) au niveau de la face interne (4a),
au moins certains desdits éléments de renfort (7) étant reliés les uns aux autres autour du périmètre de la paroi latérale (4) pour définir une couche continue d'éléments de renfort définissant autour de la cavité de retenue (5) un contour fermé, ladite couche continue d'éléments de renfort (7) conjointement au sol de la paroi latérale (4) définissant une couche de renfort (9), lesdits éléments de renfort (7) étant configurés pour définir une pluralité de couches renforcées (9) placées l'une sur l'autre pour définir ladite face interne (4a).

12. Cuve selon la revendication précédente, la structure réticulaire (8) comprenant une structure monolithique, au moins partiellement souple, en matière plastique.

13. Cuve selon l'une quelconque des revendications précédentes de cuve, où la paroi latérale (4) comprend au moins une face supérieure (4b) reliée au bord libre (6) de la paroi latérale (4) s'étendant transversalement à cette dernière à l'opposé par rapport à la base (3) le long de tout le périmètre de la paroi latérale (4) elle-même ;
et où la paroi latérale (4) comprend une face externe (4c) reliée à la face supérieure (4b) et émergeant transversalement par rapport à cette dernière, ladite face externe (4c) émergeant en approchant de la base (3) et à l'opposé par rapport à la face interne (4a), la face supérieure (4b) étant interposée entre la face interne (4a) et la face externe (4c).

14. Cuve selon la revendication précédente, dans laquelle les seconds éléments (8b) de l'élément de renfort (7), après avoir été formés, sont étirés le long de leur sens de développement prévalent, lesdits premiers éléments (8a) ne sont pas étirés ou ne présentent pas de rapport d'étirage inférieur à, éventuellement au moins la moitié, de l'un des seconds éléments (8b), le rapport d'étirage d'un élément étant défini comme le rapport de la longueur finale de l'élément lui-même une fois qu'il a été étiré jusqu'à la longueur de départ de l'élément lui-même avant d'avoir été étiré ;
et où les seconds éléments (8b) présentent un rapport d'étirage supérieur à 3, éventuellement compris entre 4 et 10, plus facultativement compris entre 5 et 8, le rapport d'étirage des seconds éléments étant défini comme le rapport d'une longueur finale des seconds éléments après les avoir étirés jusqu'à la longueur de départ des seconds éléments avant l'étirage.

15. Cuve selon l'une quelconque des revendications précédentes de cuve, où chaque élément de renfort (7) est placé selon une configuration tridimensionnelle convenant pour définir, le long d'une vue transversale de paroi latérale (4), un profil sensiblement en « C » ayant une concavité faisant face au côté opposé de la face interne (4a) de la paroi latérale (4) ;
et où l'élément de renfort de contour ouvert (7) présente au moins un premier segment (13), second segment (14) et troisième segment (15), lesdits premier et troisième segment (13; 15) étant espacés de, et sensiblement parallèles l'un à l'autre, ledit second segment (14) liant lesdits premier et troisième segment (13 ; 15) et étant transversal par rapport à ces derniers, particulièrement lesdites secondes longueurs (14) s'étendant sensiblement parallèles à la face interne (4a) de la paroi latérale (4).

16. Plante de production d'un biogaz (100), comprenant :
- au moins un premier circuit (101) se développant entre un orifice d'entrée (101a) et un orifice de sortie (101b), ledit premier circuit (101) étant configuré pour permettre à une matière organique de circuler depuis ledit orifice d'entrée (101a) vers ledit orifice de sortie (101b) ;
- au moins une cuve (1) selon l'une quelconque des revendications précédentes de cuve, ladite cuve (1) se trouvant en communication fluidique avec le premier circuit (101) et configurée pour permettre de digérer et/ou de stocker une quantité prédéterminée de matière organique, ladite cuve (1) étant configurée pour générer un biogaz suite à une fermentation bactérienne anaérobie de la matière organique ;
- au moins un second circuit (102) en communication fluidique avec ladite cuve (1) et configuré pour permettre la circulation du biogaz généré depuis ladite cuve.
